# EUROPEAN PATENT SPECIFICATION

(11) **EP 4 167 872 B1**
(45) Date of publication and mention of the grant of the patent: **22.04.2026**
(21) Application number: 22729801.5
(22) Date of filing: 25.05.2022
(51) Int. Cl.: A61B 17/072

(54) **STAPLING INSTRUMENT COMPRISING JAW MOUNTS**
KLAMMERINSTRUMENT MIT BACKENHALTERUNGEN
INSTRUMENT D'AGRAFAGE COMPRENANT DES SUPPORTS DE MÂCHOIRE

(30) Priority: 28.05.2021 US 202163194621 P; 18.08.2021 US 202163234396 P; 25.04.2022 US 202217728089
(43) Date of publication of application: 26.04.2023
(73) Proprietor: Cilag GmbH International, 6300 Zug (CH)
(72) Inventor: HUANG, Zhifan F., Cincinnati, Ohio 45242 (US); BOUDREAUX, Chad P., Cincinnati, Ohio 45242 (US)
(74) Representative: Carpmaels & Ransford LLP
(86) International application number: PCT/IB2022/054887
(87) International publication number: WO 2022/249086

(56) References cited:
- US-A1- 2004 267 311
- US-A1- 2015 173 755
- US-A1- 2018 168 642
- US-A1- 2019 000 456
- US-A1- 2020 113 563
- US-B1- 6 716 232

## Description

### BACKGROUND

The present invention relates to surgical instruments and, in various arrangements, to surgical stapling and cutting instruments and staple cartridges for use therewith that are designed to staple and cut tissue.

US 2020/113563 A1 provides a surgical fastener applying apparatus for applying fasteners to body tissue. The apparatus includes a cartridge receiving half-section defining an elongated channel member configured to releasably receive a firing assembly and a single use loading unit. A lockout structure prevents insertion of the single use loading unit into the channel member after the firing assembly is mounted to the cartridge receiving half-section
US 2019/000456 provides a surgical tool assembly that has a first jaw and a second jaw that is movable relative to the first jaw. A firing member assembly is configured to move distally from a starting position. The firing member assembly includes a first firing element and a second firing element that is configured to move between a locked position wherein the second firing element is in locking engagement with a lockout portion of the first jaw to prevent the firing member assembly from moving distally from the starting position upon application of a firing motion thereto and an unlocked position.

### SUMMARY

The present invention provides a surgical stapling assembly according to claim 1. Optional features are recited in the dependent claim. Aspects, embodiments, examples and methods of the present disclosure which are not claimed per se, are provided for illustrative purposes and are considered useful for giving context to the invention.

### BRIEF DESCRIPTION OF THE DRAWINGS

Various features of the embodiments described herein, together with advantages thereof, may be understood in accordance with the following description taken in conjunction with the accompanying drawings as follows:
FIG. 1 is a perspective view of a surgical instrument in accordance with at least one embodiment;
FIG. 1B is a left side elevation view of the surgical instrument of FIG. 1;
FIG. 1C is a right side elevation view of the surgical instrument of FIG. 1;
FIG. 1D is a front elevation view of the surgical instrument of FIG. 1;
FIG. 1E is a back elevation view of the surgical instrument of FIG. 1;
FIG. 1F is a plan view of the surgical instrument of FIG. 1;
FIG. 1G is a bottom view of the surgical instrument of FIG. 1;
FIG. 2A illustrates a surgical instrument in accordance with at least one embodiment including an end effector and lights positioned on opposite sides of the end effector which are illuminated to indicate the direction in which the end effector is being articulated;

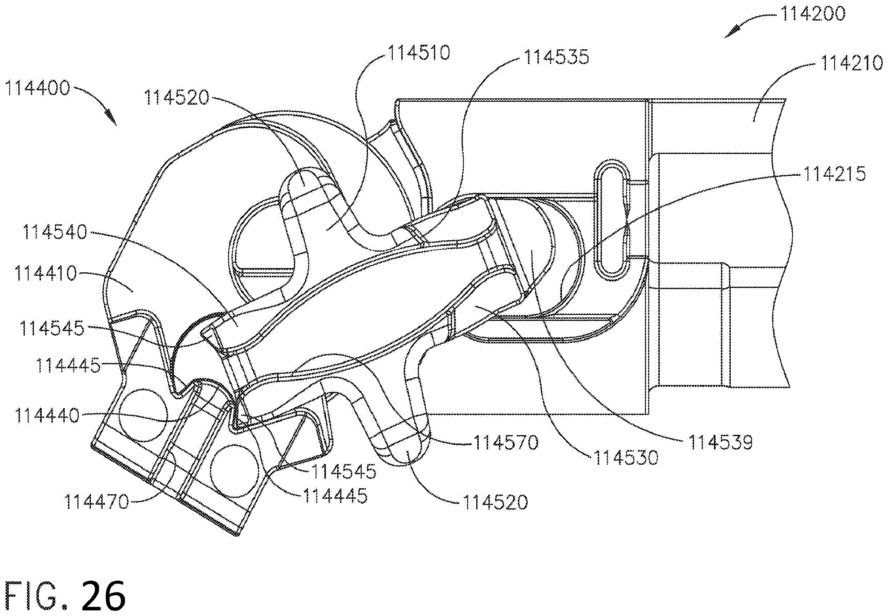

FIG. 2B is a perspective view of the surgical instrument of FIG. 26A;
FIG. 3 illustrates a surgical instrument in accordance with at least one embodiment including an articulation joint, an end effector articulatable about the articulation joint, and a translatable articulation actuator configured to rotate the end effector about the articulation joint;
FIG. 4 is a partial perspective view of an articulatable end effector, an articulation actuator configured to rotate the end effector about an articulation joint, and demarcations on the articulation actuator which indicate the direction in which an end effector is articulated and/or is being articulated;
FIG. 5 is a cross-sectional view of the end effector of the surgical instrument of FIG. 1 illustrated in an open configuration;
FIG. 6 is a partial cross-sectional view of the end effector of the surgical instrument of FIG. 1 illustrating tissue stops of the end effector;
FIG. 7 is a partial cross-sectional view of the end effector of the surgical instrument of FIG. 1 illustrating a pivot joint between a staple cartridge jaw and an anvil jaw of the end effector;
FIG. 8 is a partial plan view of the staple cartridge jaw of FIG. 7 without a staple cartridge positioned in the staple cartridge jaw;
FIG. 9 is a partial perspective view of the anvil jaw of FIG. 7;
FIG. 10 is a partial top view of the pivot joint of FIG. 7;
FIG. 11 is a partial cross-sectional view of a staple cartridge jaw of an end effector in accordance with at least one embodiment illustrated without a staple cartridge in the staple cartridge jaw;
FIG. 12A is a partial cross-sectional view of the end effector of FIG. 11 in an open configuration;
FIG. 12B is a partial cross-sectional view of the end effector of FIG. 11 in a closed configuration;
FIG. 13 is a partial cross-sectional view of the end effector of the surgical instrument of FIG. 1 illustrating a firing member in an unfired position;
FIG. 14 is a partial cross-sectional view of the end effector of the surgical instrument of FIG. 1 illustrating a cartridge stop on the anvil jaw configured to stop the proximal insertion of a staple cartridge into the staple cartridge jaw;
FIG. 15 is a partial perspective view of the anvil jaw of the surgical instrument of FIG. 1 illustrating surfaces configured to control the position of the firing member of FIG. 13 in its unfired position while the end effector is in an open configuration;
FIG16is a partial elevational view of an end effector comprising an anvil jaw and a cartridge jaw, wherein the anvil jaw comprises a distal portion that rotatable between a first operational orientation and a second operational orientation which is different than the first operational orientation, and wherein the distal portion of the anvil jaw is illustrated in the first operational orientation;
FIG. 17 is a partial perspective view of the anvil jaw of FIG. 16, wherein the distal portion of the anvil jaw is illustrated in a partially rotated orientation;
FIG. 17A depicts a connector holding the distal portion to the anvil jaw of FIG. 16;
FIG. 18 is a partial elevational view of the end effector of FIG. 16, wherein the distal portion of the anvil jaw is illustrated in the second operational orientation;
FIG. 19 is a partial perspective view of the end effector of FIG. 16, wherein the distal portion of the anvil jaw is illustrated in the second operational orientation;
FIG. 20 is a partial elevational view of an end effector of the surgical instrument illustrated in a fully-clamped configuration;
FIG. 21 is a partial elevational view of an end effector of in an open configuration;
FIG. 22 is a cross-sectional view of an end effector of illustrated in a partially-closed configuration;
FIG. 23 is a partial cross-sectional view of a channel of the end effector of FIG. 20;

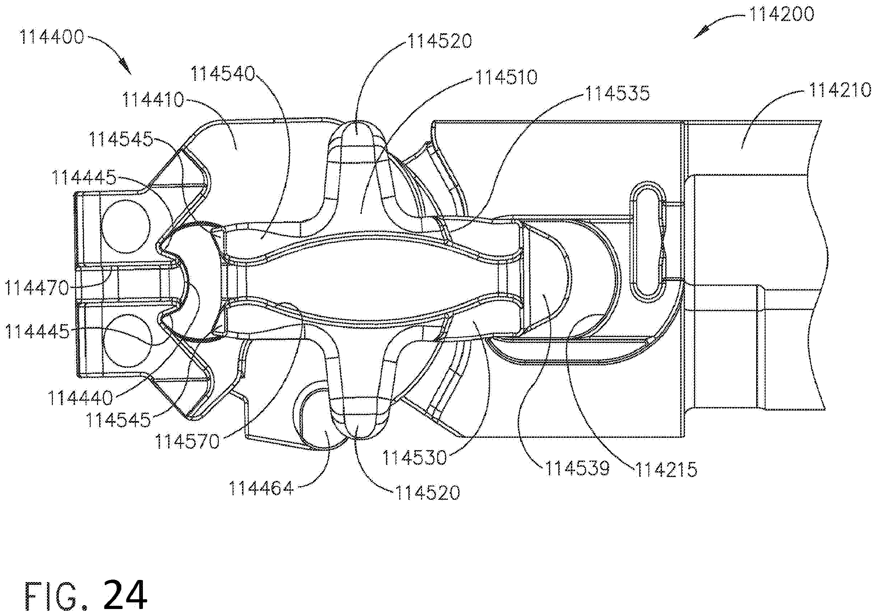

FIG. 24 is a plan view of an end effector of a surgical instrument illustrated in an unarticulated position;

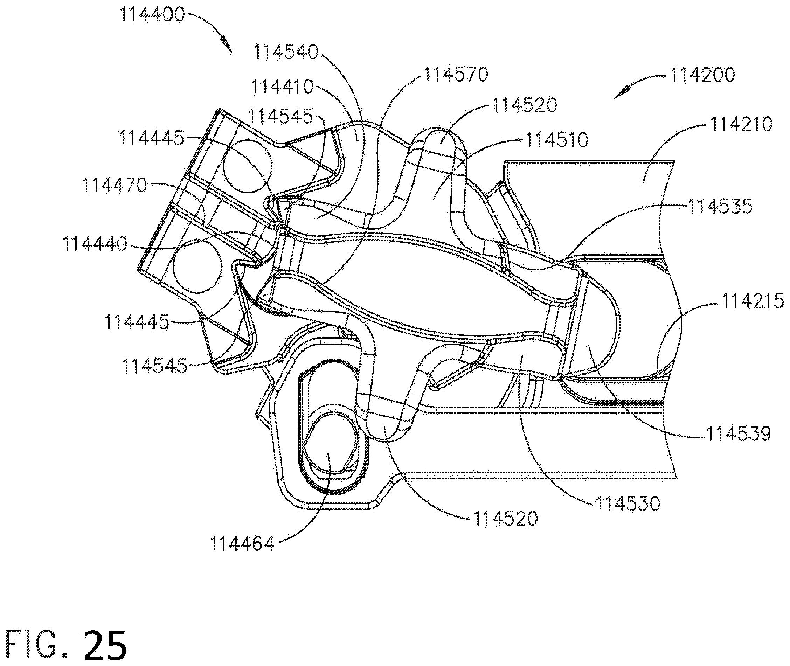

FIG. 25 illustrates the end effector of FIG. 24 articulated in a first direction;
FIG. 26 illustrates the end effector of FIG. 24 articulated in a second direction;

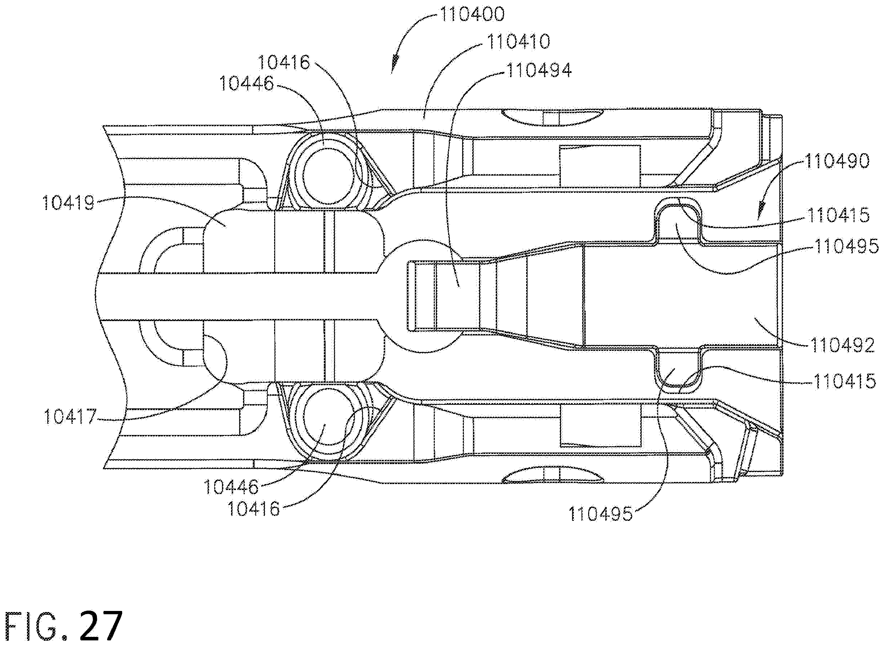

FIG. 27 is a partial plan view of a jaw of the end effector of a surgical instrument including a staple firing lockout;
FIG. 28 is a partial elevational view of a staple firing system and the staple firing lockout of FIG. 27;
FIG. 29 is an exploded view of the articulation joint of a surgical instrument;

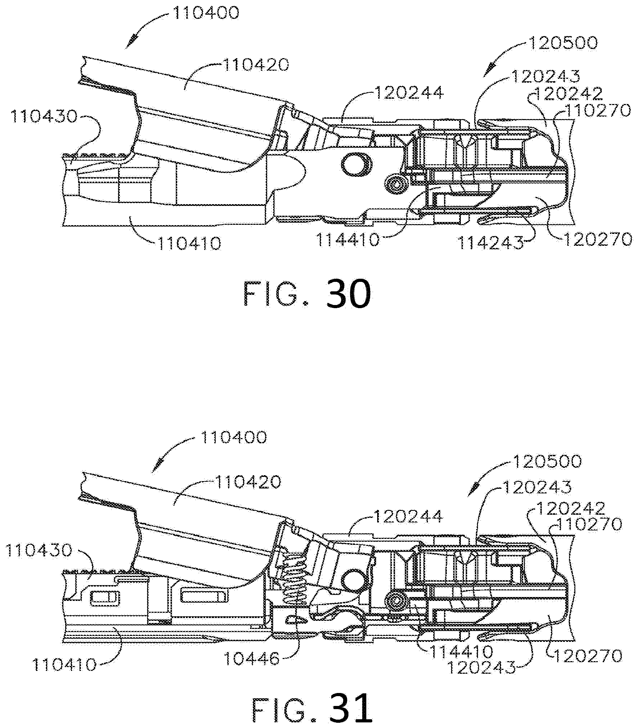

FIG. 30 is a partial cross-sectional elevational view of a surgical instrument illustrating the end effector in an open configuration;
FIG. 31 is a partial cross-sectional elevational view of a surgical instrument illustrated with some components removed;
FIG. 32 is a perspective view of an end effector of a surgical instrument in accordance with at least one embodiment; FIG. 33 is an exploded view of the end effector of FIG. 32;
FIG. 34 is another exploded view of the end effector of FIG. 32;
FIG. 35 is a partial plan view of the end effector of FIG. 32;
FIG. 36 is a perspective view of an end effector of a surgical instrument in accordance with at least one embodiment;
FIG. 37 is an exploded view of the end effector of FIG. 36;
FIG. 38 is a partial cross-sectional view of the end effector of FIG. 36;
FIG. 39 is a perspective view of an end effector of a surgical instrument in accordance with at least one embodiment illustrated without a staple cartridge seated in a cartridge jaw of the end effector;
FIG. 40 is a partial cross-sectional view of the end effector of FIG. 39 illustrated in an open configuration with a staple cartridge seated in the cartridge jaw;
FIG. 41 is a partial cross-sectional view of the end effector of FIG. 39 illustrated in a clamped configuration;

Corresponding reference characters indicate corresponding parts throughout the several views.

### DETAILED DESCRIPTION

Numerous specific details are set forth to provide a thorough understanding of the overall structure, function, manufacture, and use of the embodiments as described in the specification and illustrated in the accompanying drawings. Well-known operations, components, and elements have not been described in detail so as not to obscure the embodiments described in the specification. The reader will understand that the embodiments described and illustrated herein are non-limiting examples, and thus it can be appreciated that the specific structural and functional details disclosed herein may be representative and illustrative. Variations and changes thereto may be made without departing from the scope of the claims.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a surgical system, device, or apparatus that "comprises," "has," "includes" or "contains" one or more elements possesses those one or more elements, but is not limited to possessing only those one or more elements. Likewise, an element of a system, device, or apparatus that "comprises," "has," "includes" or "contains" one or more features possesses those one or more features, but is not limited to possessing only those one or more features.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary devices and methods are provided for performing laparoscopic and minimally invasive surgical procedures. However, the reader will readily appreciate that the various methods and devices disclosed herein can be used in numerous surgical procedures and applications including, for example, in connection with open surgical procedures. As the present Detailed Description proceeds, the reader will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device that has a working channel through which the end effector and elongate shaft of a surgical instrument can be advanced.

A surgical stapling system can comprise a shaft and an end effector extending from the shaft. The end effector comprises a first jaw and a second jaw. The first jaw comprises a staple cartridge. The staple cartridge is insertable into and removable from the first jaw; however, other embodiments are envisioned in which a staple cartridge is not removable from, or at least readily replaceable from, the first jaw. The second jaw comprises an anvil configured to deform staples ejected from the staple cartridge. The second jaw is pivotable relative to the first jaw about a closure axis; however, other embodiments are envisioned in which the first jaw is pivotable relative to the second jaw. The surgical stapling system further comprises an articulation joint configured to permit the end effector to be rotated, or articulated, relative to the shaft. The end effector is rotatable about an articulation axis extending through the articulation joint. Other embodiments are envisioned which do not include an articulation joint.

The staple cartridge comprises a cartridge body. The cartridge body includes a proximal end, a distal end, and a deck extending between the proximal end and the distal end. In use, the staple cartridge is positioned on a first side of the tissue to be stapled and the anvil is positioned on a second side of the tissue. The anvil is moved toward the staple cartridge to compress and clamp the tissue against the deck. Thereafter, staples removably stored in the cartridge body can be deployed into the tissue. The cartridge body includes staple cavities defined therein wherein staples are removably stored in the staple cavities. The staple cavities are arranged in six longitudinal rows. Three rows of staple cavities are positioned on a first side of a longitudinal slot and three rows of staple cavities are positioned on a second side of the longitudinal slot. Other arrangements of staple cavities and staples may be possible.

The staples are supported by staple drivers in the cartridge body. The drivers are movable between a first, or unfired position, and a second, or fired, position to eject the staples from the staple cavities. The drivers are retained in the cartridge body by a retainer which extends around the bottom of the cartridge body and includes resilient members configured to grip the cartridge body and hold the retainer to the cartridge body. The drivers are movable between their unfired positions and their fired positions by a sled. The sled is movable between a proximal position adjacent the proximal end and a distal position adjacent the distal end. The sled comprises a plurality of ramped surfaces configured to slide under the drivers and lift the drivers, and the staples supported thereon, toward the anvil.

Further to the above, the sled is moved distally by a firing member. The firing member is configured to contact the sled and push the sled toward the distal end. The longitudinal slot defined in the cartridge body is configured to receive the firing member. The anvil also includes a slot configured to receive the firing member. The firing member further comprises a first cam which engages the first jaw and a second cam which engages the second jaw. As the firing member is advanced distally, the first cam and the second cam can control the distance, or tissue gap, between the deck of the staple cartridge and the anvil. The firing member also comprises a knife configured to incise the tissue captured intermediate the staple cartridge and the anvil. It is desirable for the knife to be positioned at least partially proximal to the ramped surfaces such that the staples are ejected ahead of the knife.

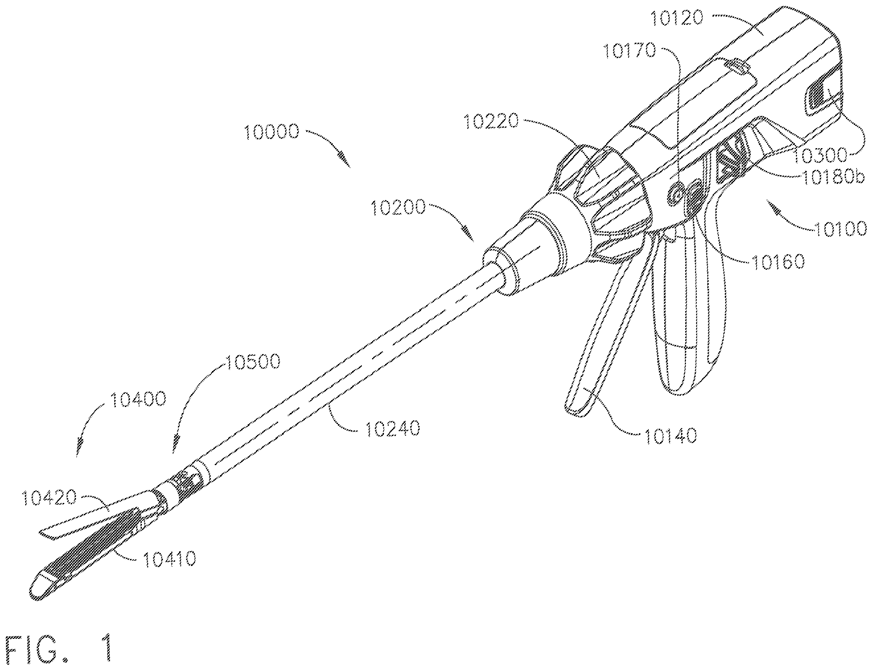

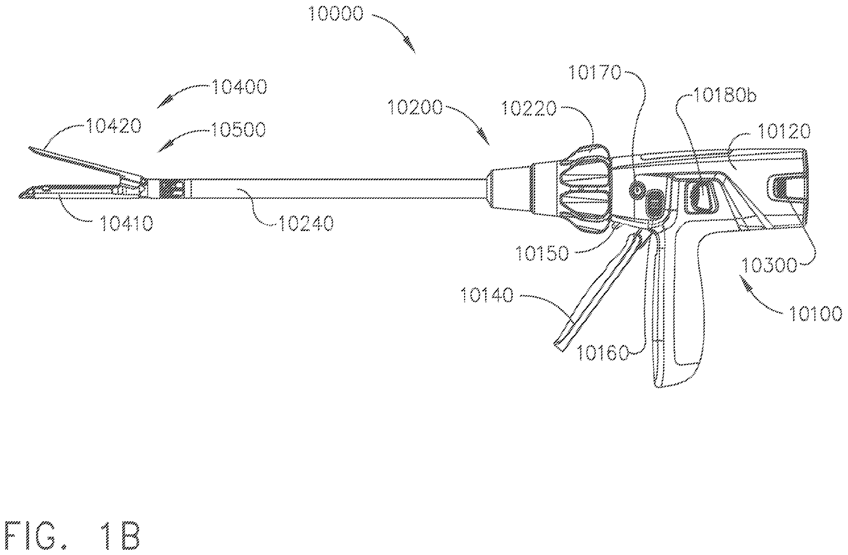

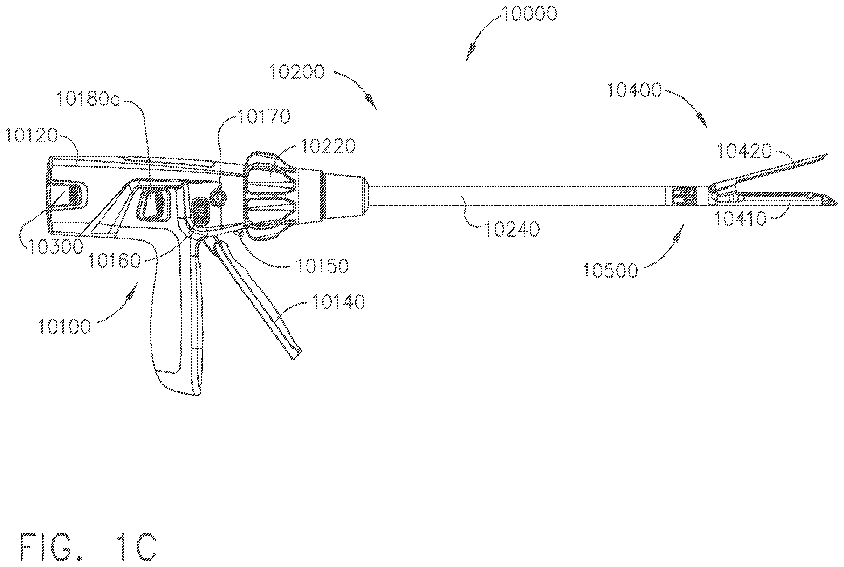

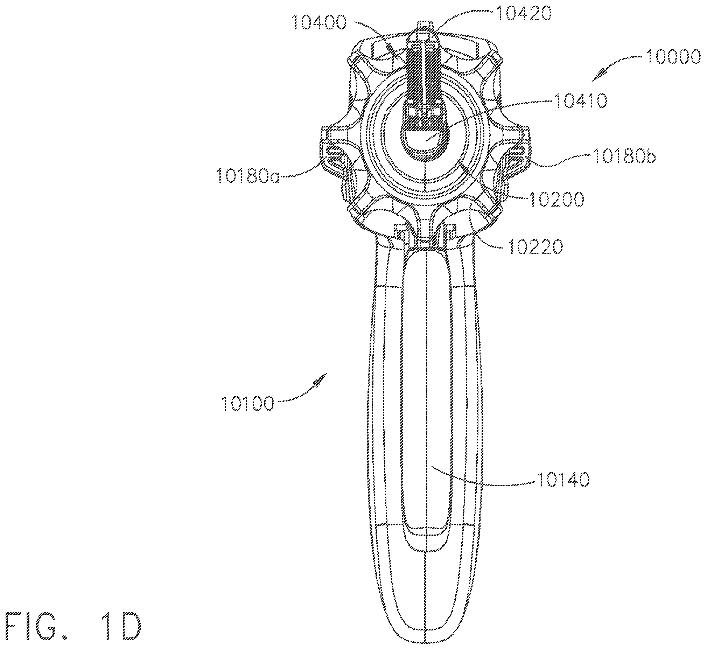

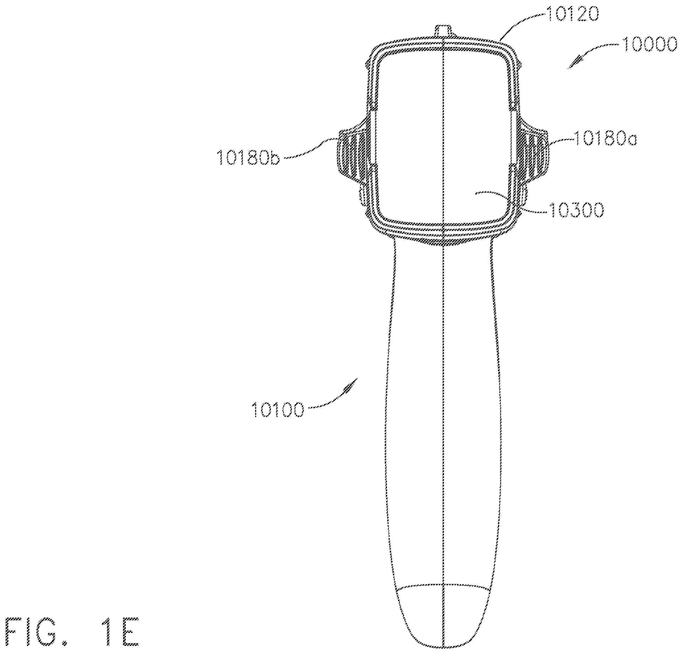

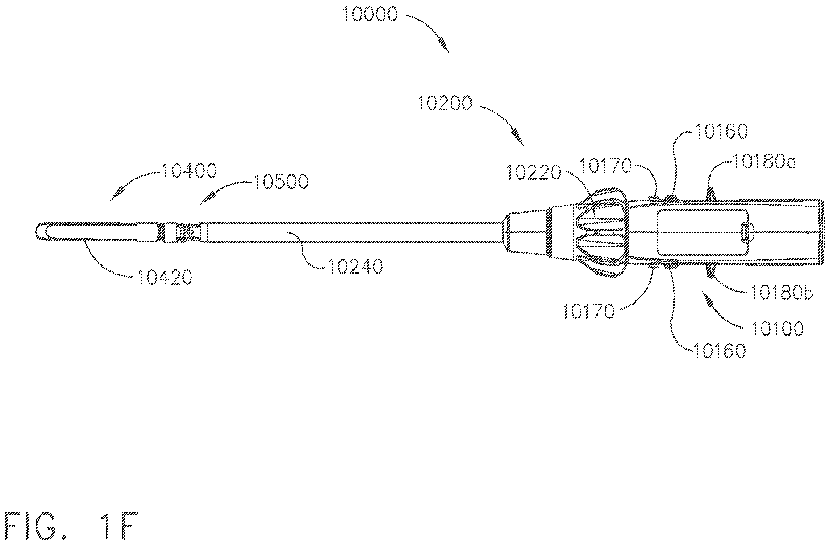

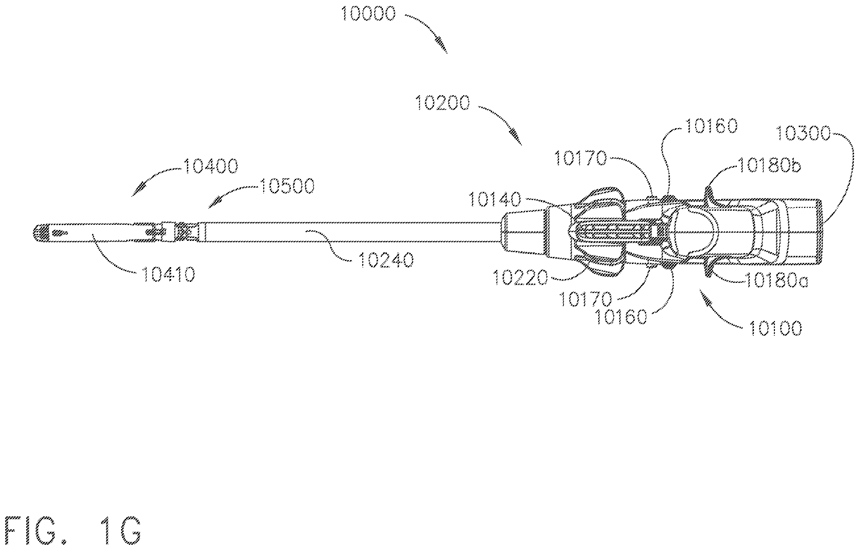

A surgical instrument 10000 is illustrated in FIG. 1. The surgical instrument 10000 comprises a handle 10100, a shaft 10200 extending from the handle 10100, and an end effector 10400. The end effector 10400 comprises a first jaw 10410 configured to receive a staple cartridge and a second jaw 10420 movable relative to the first jaw 10410. The second jaw 10420 comprises an anvil including staple forming pockets defined therein. The surgical instrument 10000 further comprises a closure actuator 10140 configured to drive a closure system of the surgical instrument 10000 and move the second jaw 10420 between an unclamped position and a clamped position.

The end effector 10400 is attached to the shaft 10200 about an articulation joint 10500 and is rotatable within a plane about an articulation axis. The shaft 10200 defines a longitudinal axis and the end effector 10400 is articulatable between a position in which the end effector 10400 is aligned with the longitudinal axis and positions in which the end effector 10400 extends at a transverse angle relative to the longitudinal axis. The handle 10100 comprises an electric motor and a control system configured to control the operation of the electric motor. The electric motor comprises a brushless DC motor; however, the electric motor can comprise any suitable motor, such as a brushed DC motor, for example. The handle 10100 further comprises a replaceable and/or rechargeable battery 10300 attachable to the handle housing which powers the surgical instrument 10000. The electric motor is operably coupled with a firing drive 10250 of the surgical instrument 10000 and is configured to drive a firing member of the firing drive 10250 through a staple firing stroke. The electric motor comprises a rotatable output including a gear engaged with a translatable rack of the firing drive 10250. The electric motor is operated in a first direction to drive the firing member through the staple firing stroke and a second, or opposite, direction to retract the firing member and/or reset the firing drive 10250. The surgical instrument 10000 further comprises an actuator 10150 in communication with the motor control system which, when actuated or rotated, signals to the motor control system to operate the electric motor in the first direction and begin the staple firing stroke. If the actuator 10150 is released, the motor control system stops the electric motor. When the actuator 10150 is re-actuated, the motor control system operates the electric motor in the first direction once again to continue the staple firing stroke. When the firing member reaches the end of the staple firing stroke, the control system stops the electric motor awaiting input from the clinician. When the clinician releases the actuator 10150 at such point, the control system reverses the operation of the electric motor to retract the firing member back into its unfired position. The handle 10100 further comprises a retraction actuator in communication with the motor control system that reverses the direction of the electric motor to retract the firing drive when actuated by the clinician. When the retraction actuator is depressed, the staple firing stroke is terminated regardless of whether the firing member had reached the end of the staple firing stroke.

The electric motor of the surgical instrument 10000 is also used to selectively drive an articulation drive system to articulate the end effector 10400. More specifically, the articulation drive system comprises an articulation driver that is selectively engageable with the firing drive and, when the articulation driver is engaged with the firing drive, the articulation driver is movable proximally and distally by the operation of the electric motor to articulate the end effector 10400. When the electric motor is operated in its first direction, in such instances, the end effector 10400 is articulated in a first direction to push the articulation driver distally. Similarly, the end effector 10400 is articulated in a second direction when the electric motor is operated in its second direction to pull the articulation driver proximally. When the articulation driver is not engaged with the firing drive, the operation of the electric motor does not articulate the end effector 10400. Instead, in such instances, the electric motor only moves the firing drive. That said, it should be appreciated that the movement of the firing drive to articulate the end effector 10400 does not cause the staple firing stroke to be performed. The range of motion needed to articulate the end effector 10400 is small, as compared to the range of motion of the staple firing stroke, and occurs proximal to the beginning of the staple firing stroke such that the staples are not ejected and the tissue is not cut while the end effector 10400 is being articulated. The surgical instrument 10000 further comprises an articulation lock which unlocks when the articulation driver is moved longitudinally by the firing drive and then locks the end effector 10400 in position when the articulation driver is not being driven by the firing drive. The above being said, a surgical instrument can comprise a separate articulation motor in addition to the firing motor for driving the articulation drive system.

Further to the above, the shaft 10200 is rotatable relative to the handle 10100. The shaft 10200 comprises a frame 10210 attached to the frame 10110 of the handle 10100. In embodiments where the shaft 10200 is readily removable from the handle 10100, the shaft frame 10210 can detach from the handle frame 10110. In embodiments where the shaft 10200 is not removable from the handle 10100, the shaft frame 10210 and the handle frame 10110 can be integrally formed. In any event, the shaft 10200 comprises a nozzle, or grip, 10220 fixedly mounted to the closure tube 10240 of the shaft 10200. The grip 10220 comprises finger grooves 10222 defined therein and ridges 10224 extending between the finger grooves 10222 that provide walls against which a clinician can push their finger and assist the clinician in rotating the shaft 10200 about its longitudinal axis.

Notably, further to the above, the end effector 10400 rotates with the shaft 10200 when the shaft 10200 is rotated about its longitudinal axis. Thus, the end effector 10400 rotates clockwise when the shaft 10200 is rotated clockwise by the clinician and counter-clockwise when the shaft 10200 is rotated counter-clockwise by the clinician. In various alternative embodiments, the surgical instrument 10000 comprises an electric motor configured to rotate the shaft 10200 about its longitudinal axis. In either event, the shaft 10200 is rotatable from a top-dead-center (TDC) position in which the anvil 10420 is positioned directly above the staple cartridge jaw 10410 to any other suitable position within a full 360 degree range of positions. For instance, the shaft 10200 is rotatable into a right 90 degree position in which the anvil 10420 is facing to the right of the handle 10100 or a left 90 degree position in which the anvil 10420 is facing to the left of the handle 10100. The shaft 10200 is also rotatable into a bottom-dead-center (BDC) position in which the staple cartridge jaw 10410 is positioned directly above the anvil 10420.

As described above, the end effector 10400 is both articulatable about the articulation joint 10500 and rotatable with the shaft 10200. When the end effector 10400 is rotated in a plane when the end effector 10400 is in its TDC position, as mentioned above, the articulation control 10160 is intuitive to the user - push up to articulate left and push down to articulate right. This arrangement is also intuitive even after the shaft 10200 - and end effector 10400 - have been rotated 90 degrees to the right or to the left. However, when the shaft 10200 and end effector 10400 have been rotated past 90 degrees in either direction, the articulation control 10160 can become counter-intuitive to the clinician. In fact, the articulation control 10160 can seem backwards. With this in mind, the control system of the surgical instrument 10000 is configured to flip the manner in which the surgical instrument responds to the articulation control 10160 when the shaft 10200 and end effector 10400 have been rotated past 90 degrees in either direction. In such instances, the controls become: push up to articulate right and push down to articulate left. To this end, as described in greater detail below, the surgical instrument 10000 is configured to detect the orientation of the shaft 10200 relative to the handle 10100, i.e., it is configured to detect whether the end effector 10400 is at least partially upside down with respect to the handle 10100 and then enter an alternative operational control mode in which the responsiveness of the surgical instrument 10000 to the articulation control 10160 has been reversed. Such an arrangement can make the surgical instrument 10000 easier to use in various instances.

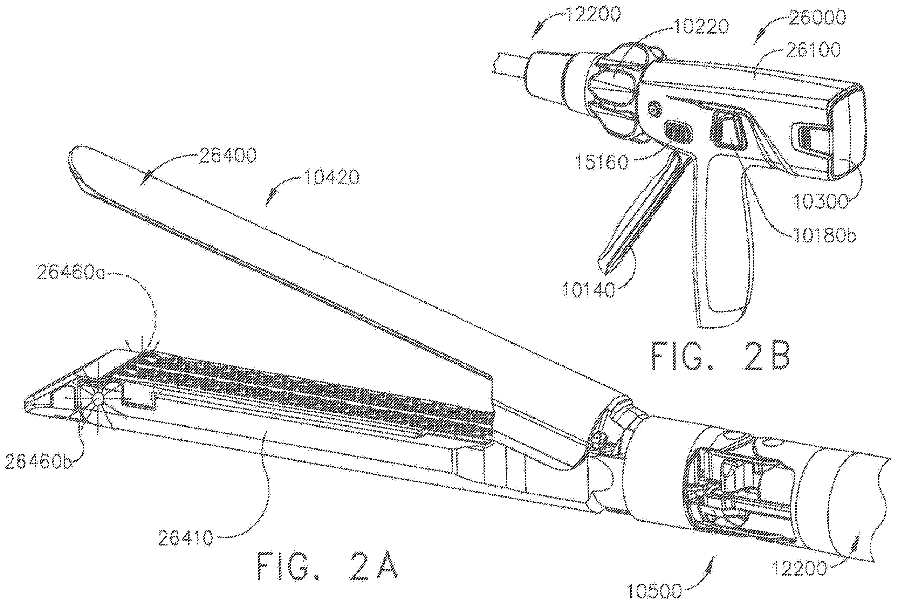

A surgical instrument 26000 is illustrated in FIGS. 2A and 2B. The surgical instrument 26000 comprises a handle 26100 and a shaft 12200 extending from the handle 26100. The shaft 12200 comprises an end effector 26400 including a staple cartridge jaw 26410 and an anvil jaw 10420. The end effector 26400 further comprises a first articulation indicator light 26460a positioned on a first side of the end effector 26400 and a second articulation indicator light 26460b positioned on a second side of the end effector 26400. Similar to the above, the control system of the surgical instrument 26000 illuminates the first articulation indicator light 26460a when the end effector 26400 is articulated in the first direction. In such instances, the control system does not illuminate the second articulation indicator light 26460b. Correspondingly, the control system of the surgical instrument 26000 illuminates the second articulation indicator light 26460b when the end effector 26400 is articulated in the second direction. In such instances, the control system does not illuminate the first articulation indicator light 26460a. The indicator lights 26460a and 26460b are mounted to and/or embedded in the frame of the staple cartridge jaw 26410. That said, the indicator lights 26460a and 26460b can be mounted to and/or embedded in the staple cartridge positioned in the staple cartridge jaw 26410. In such instances, the staple cartridge jaw 26410 comprises an electrical circuit in communication with the control system of the surgical instrument that is placed in communication with an electrical circuit in the staple cartridge when the staple cartridge is seated in the staple cartridge jaw 26410.

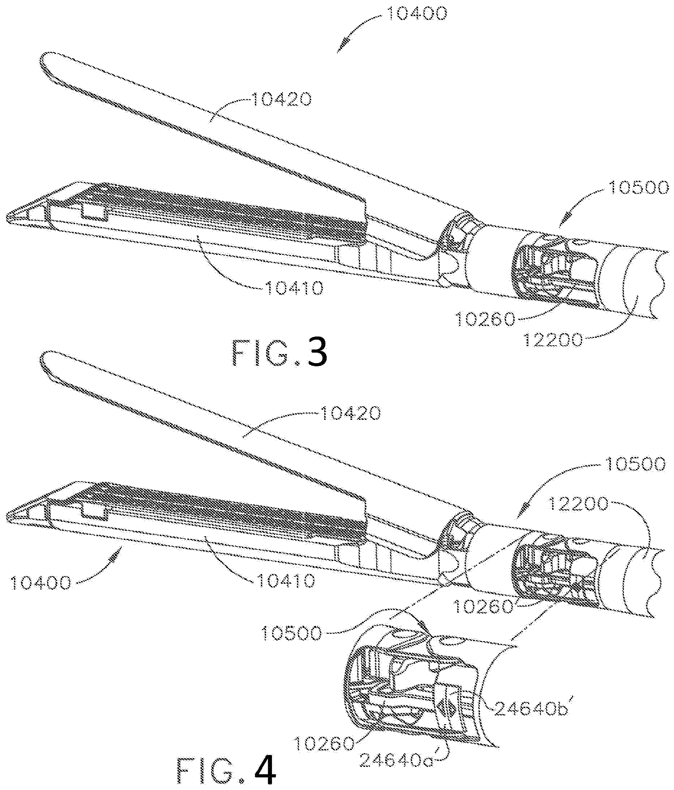

As discussed above, the articulation system of a surgical instrument can include an articulation driver which is movable proximally to articulate the end effector in a first direction and distally to articulate the end effector in a second direction. Referring to FIG. 3, a surgical instrument can comprise a handle 26100, a shaft 12200 extending from the handle 26100, and an end effector 10400 rotatably connected to the shaft 12200 about an articulation joint 10500. The shaft 12200 comprises an articulation driver 10260 comprising a proximal end operably coupled to an articulation drive system and a distal end coupled to the end effector 10400. To this end, the articulation driver 10260 extends distally past the articulation joint 10500 and, in this embodiment, is partially visible to a clinician holding the surgical instrument. The portion of the articulation driver 10260 visible to the clinician is also visible to the clinician through an endoscope monitor. In fact, a clinician may be able to observe the motion of the articulation driver 10260 through the endoscope monitor. The visible portion of the articulation driver 10260 comprises indicia, such as indicia 24640a' and 24640b', for example, thereon which correlates the movement of the articulation driver 10260 to the movement of the end effector 10400. In at least one instance, the indicia can comprise a first set of indicia which includes a distally-directed arrow 24640a' and a circular arrow indicating the direction that the end effector 10400 will be rotated if the articulation driver 10260 is moved distally. The indicia can also comprises a second set of indicia which includes a proximally-directed arrow 24640b' and a circular arrow in the opposite direction indicating the direction that the end effector 10400 will be rotated if the articulation driver 10260 is moved proximally. An alternative articulation driver 10260' is illustrated in FIG. 4 that comprises a laterally-extending portion which can be readily visible to the clinician. In such instances, the above-discussed indicia is positioned on the laterally-extending portion.

As discussed above, an end effector of a surgical instrument can be rotatable in more than one direction and/or plane. To achieve this, in various embodiments, a surgical instrument comprises a first motor-driven system for moving the end effector in a left-to-right manner and a second motor-driven system for moving the end effector in an up-to-down manner. Both motor-driven systems are in communication with the control system of the surgical instrument and are drivable sequentially and/or concurrently by the control system to position the end effector in the direction indicated by the input from the articulation actuator, or articulation actuators.

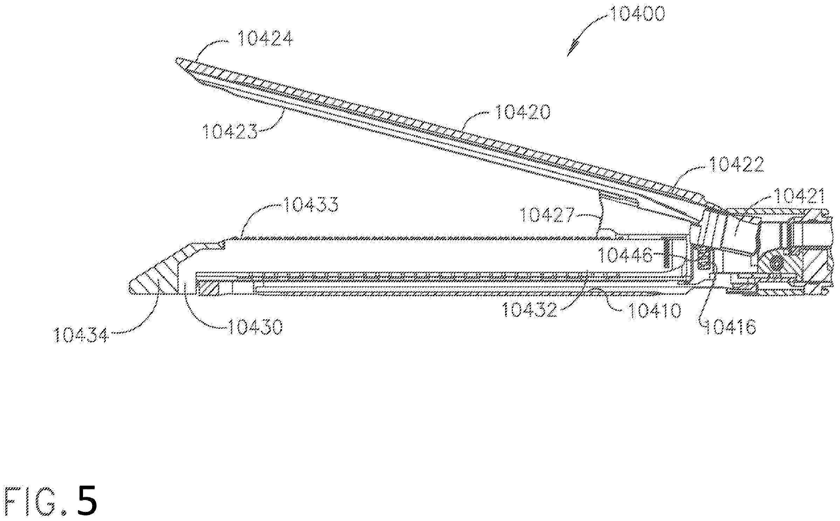

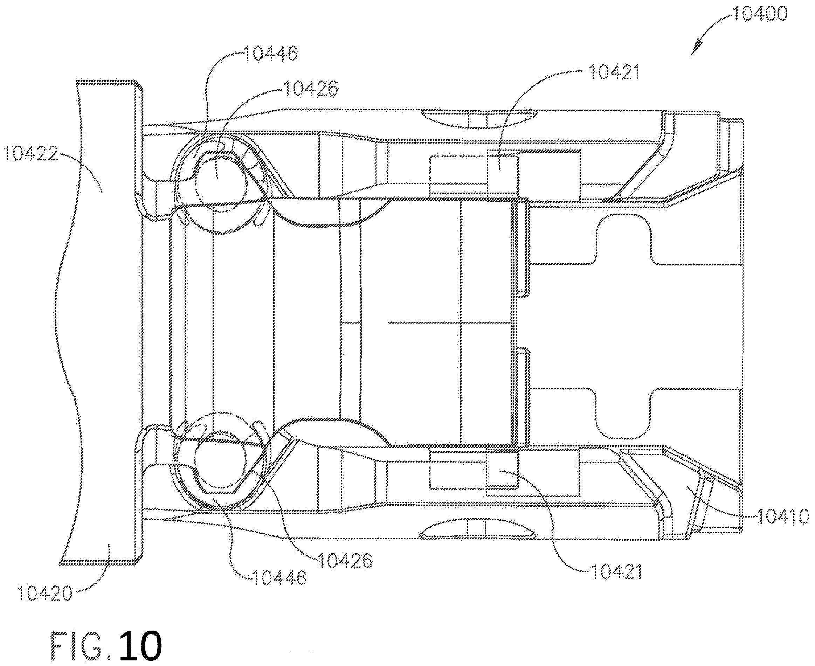

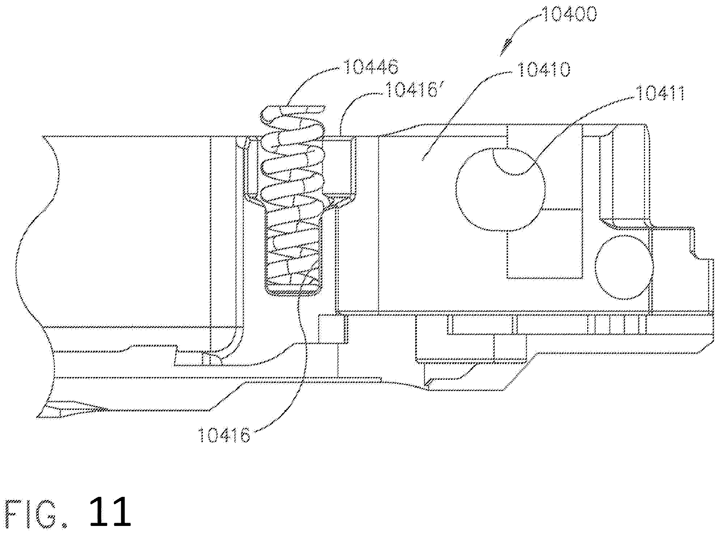

The end effector 10400 comprises a staple cartridge jaw 10410 configured to receive a replaceable staple cartridge, such as staple cartridge 10430, for example, and an anvil jaw 10420 configured to deform the staples ejected from the staple cartridge 10430. The staple cartridge jaw 10410 comprises a channel including a bottom support and two lateral sidewalls extending upwardly configured to receive the staple cartridge 10430. The staple cartridge 10430 comprises a proximal end 10432, a distal end 10434, and a deck 10433 extending between the proximal end 10432 and the distal end 10434. When the staple cartridge 10430 is inserted into the staple cartridge jaw 10410, the proximal end 10432 is guided into position between the staple cartridge jaw 10410 and the anvil jaw 10420 and then seated into the staple cartridge jaw 10410. The anvil jaw 10420 comprises a proximal end 10422, a distal end 10424, a tissue compression surface 10423 extending between the proximal end 10422 and the distal end 10424, and a pivot 10421 rotatably connecting the anvil jaw 10420 to the staple cartridge jaw 10410. Referring to FIG. 11, the anvil jaw 10420 comprises lateral pins that extend into apertures 10411 defined in the staple cartridge jaw 10410. As discussed above, the anvil jaw 10420 is rotatable into a closed, or clamped, position by the closure drive of the stapling instrument 10000. When the closure drive is retracted, the anvil jaw 10420 is opened. Referring to FIGS. 5-10, the stapling instrument 10000 further comprises one or more biasing members, or springs, 10446 configured to open the anvil jaw 10420 when the closure drive is retracted. The surgical instrument 10000 comprises two opening springs 10446, but could comprise any suitable number of biasing members. In any event, each spring 10446 is positioned in a recess 10416 defined in the staple cartridge jaw 10410. The recesses 10416 closely receive the springs 10446 such that the springs 10446 do not buckle under a compressive load; however, the recesses 10416 are sized and configured to accommodate any lateral expansion of the springs 10446 as the anvil jaw 10420 is being closed.

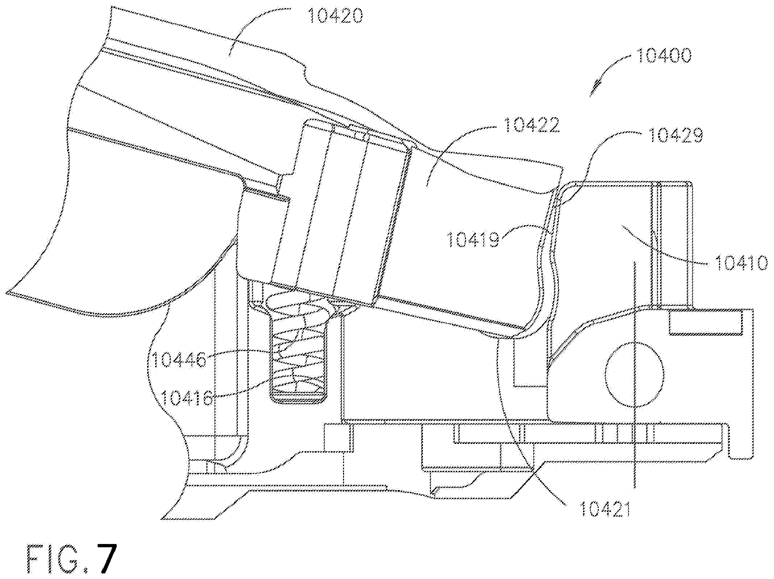

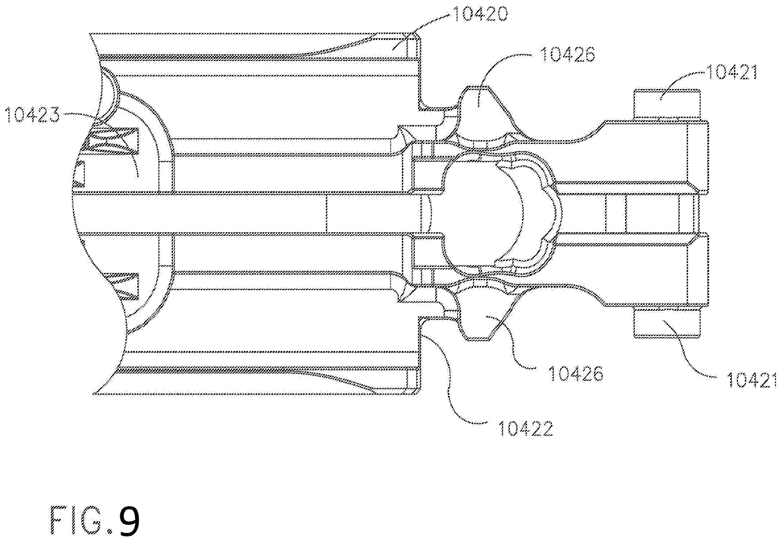

Referring primarily to FIG. 9, the anvil jaw 10420 comprises lateral tabs 10426 adjacent the proximal end 10422 of the anvil 10420 which are in contact with the springs 10446. When the anvil jaw 10420 is closed, the springs 10446 are compressed between the lateral tabs 10426 and the bottom of the recesses 10416. When the closure system is retracted, the springs 10446 resiliently re-expand and push upwardly on the lateral tabs 10426 to rotate the anvil jaw 10420 into its open, or unclamped, position. Notably, referring primarily to FIG. 7, the staple cartridge jaw 10410 has a stop portion 10419 defined thereon which is contacted by the proximal end 10422 of the anvil 10420 when the anvil 10420 reaches its fully-open position. The anvil 10420 comprises a proximal stop surface 10429 which contacts the stop portion 10419 of the staple cartridge jaw 10410. In such instances, the anvil jaw 10420 cannot be opened any further. As a result of the above, the springs 10446 hold the anvil jaw 10420 against the stop portion 10419 of the staple cartridge jaw 10410 until the anvil jaw 10420 is closed once again.

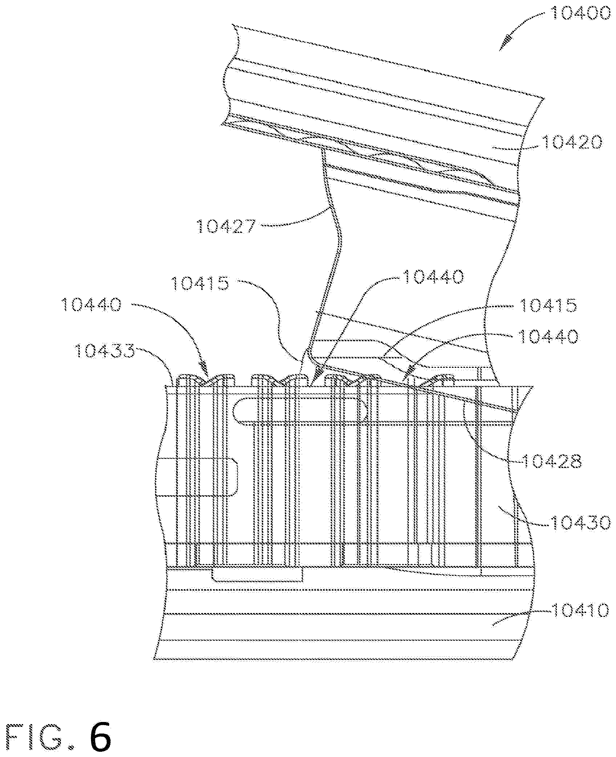

When the anvil jaw 10420 is in its open position, the staple cartridge jaw 10410 is positioned on one side of the tissue that is to be stapled and the anvil jaw 10420 is positioned on the opposite side. In such instances, the end effector 10400 is moved relative to the tissue until the tissue is suitably positioned between the staple cartridge jaw 10410 and the anvil jaw 10420. The anvil jaw 10420 comprises lateral tissue stops 10427 which extend downwardly alongside the staple cartridge jaw 10410 which are configured to make sure that the tissue positioned within the end effector 10400 is positioned over the staple cavities in the staple cartridge 10430. Referring primarily to FIG. 6, the tissue stops 10427 extend distally with respect to the proximal-most staple cavities 10440. In at least one instance, the tissue stops 10427 extend distally with respect to at least one staple cavity 10440 in each longitudinal row of staple cavities 10440. As a result, the tissue stops 10427 make sure that the tissue captured in the end effector 10400 is not cut by the tissue cutting knife without being stapled. When the anvil jaw 10420 is closed, the tissue stops 10427 move relative to the staple cartridge jaw 10410. The tissue stops 10427 are sized and configured such that tissue does not become accidentally pinched between the tissue stops 10427 and the lateral sides of the staple cartridge jaw 10410. More specifically, the bottom edges 10428 of the tissue stops 10427 are configured such that they extend alongside the lateral sides of the staple cartridge jaw 10410 even when the anvil jaw 10420 is in its fully-open position, as illustrated in FIG. 6. Notably, the lateral sides 10415 of the staple cartridge jaw 10410 extend upwardly above the deck 10433 to make sure that there is overlap between the tissue stops 10427 and the lateral sides 10415 of the staple cartridge jaw 10410 - when viewed from the side - throughout the entire range of motion of the anvil jaw 10420.

In various embodiments, further to the above, the distal edges of the tissue stops 10427 extend below the deck 10433 throughout the entire range of motion of the anvil jaw 10420. Thus, the distal edges of the tissue stops 10427 extend below the top surface of the deck 10433 when the anvil jaw 10420 is in its fully-open position and its fully-clamped position. Such an arrangement reduces the possibility of the tissue being pinched when the anvil jaw 10420 is moved. In certain embodiments, the staple cartridge comprises tissue stops that extend upwardly from the deck 10433 alongside the tissue stops 10427. Similar to the above, the distal edges of the tissue stops 10427 extend below the cartridge tissue stops through the entire range of motion of the anvil jaw 10420. Such an arrangement also reduces the possibility of the tissue being pinched when the anvil jaw 10420 is moved. Moreover, these arrangements would be useful in embodiments where the staple cartridge jaw 10410 moves relative to the anvil jaw 10420.

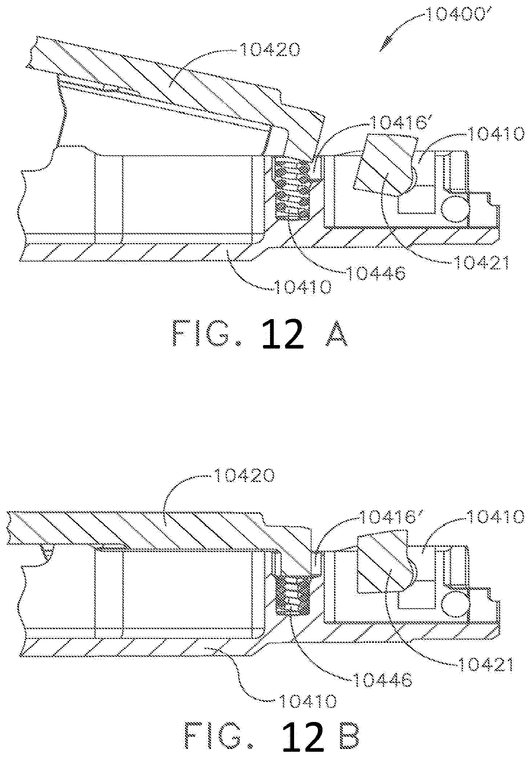

As discussed above and referring primarily to FIGS. 11, 12A, and 12B the end effector 10400 comprises a staple cartridge jaw 10410 that includes spring recesses 10416 defined therein which comprise wider top openings 10416'. The spring recesses 10416 still support the springs 10446 and keep them from buckling, but the wider top openings 10416' of the spring recesses 10416 provide clearance for the lateral tabs 10426 when the anvil jaw 10420 is in its closed position. In such an arrangement, the lateral tabs 10426 can move into the staple cartridge jaw 10410 to compress the springs 10446. In such instances, the springs 10446 can be highly compressed by the anvil jaw 10420, thereby assuring a strong opening force from the springs 10446 when the anvil jaw 10420 is released by the closure drive. The above being said, embodiments are envisioned without the wider top openings 10416'. In such embodiments, the springs are closely received by the spring recesses 10416 along the length of the springs 10446.

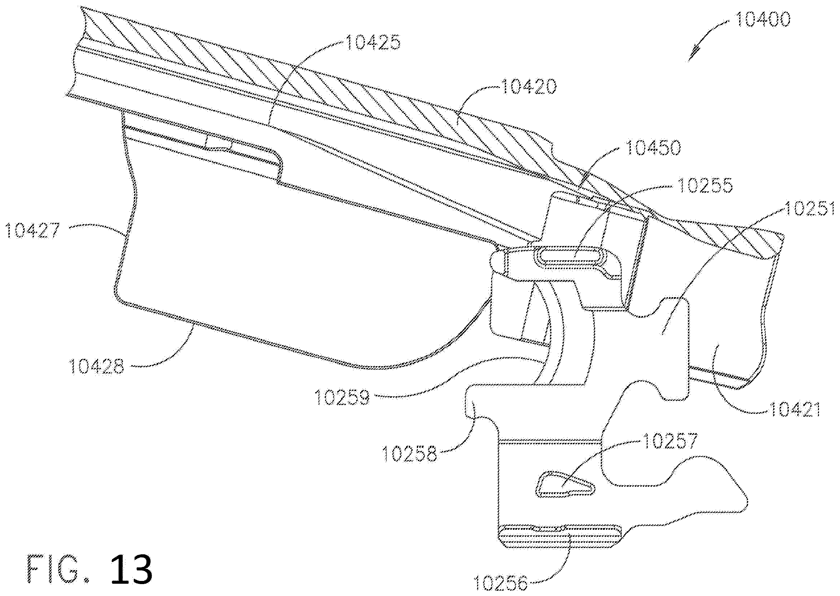

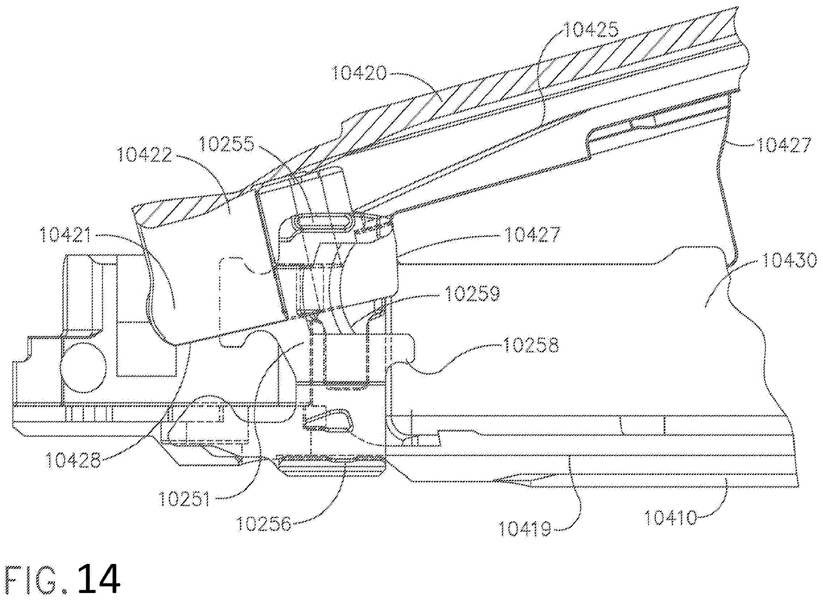

The tissue cutting member 10251 of the firing drive of the stapling instrument 10000 is illustrated in FIGS. 13 and 14, the tissue cutting member comprises a body including a distal nose 10258 and a tissue cutting edge 10259 which pass through the end effector 10400 during a staple firing stroke. The tissue cutting member 10251 further comprises a top cam member 10255 configured to engage the anvil jaw 10420 and a bottom cam member 10256 configured to engage the staple cartridge jaw 10410 during the staple firing stroke. A longitudinal cam surface 10425 in a longitudinal slot of the anvil jaw 10420 can be seen in FIG. 13 which is engaged by the top cam member 10255 during the staple firing stroke. The staple cartridge jaw 10410 also has a longitudinal cam surface 10419 which is engaged by the bottom cam member 10256. The cam members 10255 and 10256 position the jaws 10410 and 10420 relative to one another during the staple firing stroke and hold the jaws 10410 and 10420 in their closed configuration throughout the staple firing stroke. The cam members 10255 and 10256 also set the staple forming gap between the staple drivers in the staple cartridge and the forming pockets defined in the anvil jaw 10420.

Notably, FIGS. 13 and 14 illustrate the anvil jaw 10420 in its open position and the tissue cutting member 10251 in its unfired position, i.e., its position before the staple firing stroke has begun. The anvil jaw 10420 comprises a clearance pocket 10450 defined therein which is aligned with the top cam member 10255 of the tissue cutting member 10251 when the tissue cutting member 10251 is in its unfired position. Such an arrangement allows the tissue cutting member 10251 to be parked just proximal to the longitudinal cam surface 10425 in the anvil jaw 10420, and the corresponding cam surface in the staple cartridge jaw 10410, when the tissue cutting member 10251 is in its unfired position. Such an arrangement provides for a shorter, and more maneuverable, end effector for a given staple line length. Moreover, the tissue cutting member 10251 comprises a tissue cutting edge 10259 that is positioned proximally with respect to the staple cavities defined in the staple cartridge and proximally with respect to the distal edges of the tissue stops when the tissue cutting member is in its unfired position. As a result, the tissue being inserted into the end effector is unlikely to be cut by the tissue cutting edge 10259 until the tissue cutting member 10251 is advanced distally from its unfired position during a firing stroke.

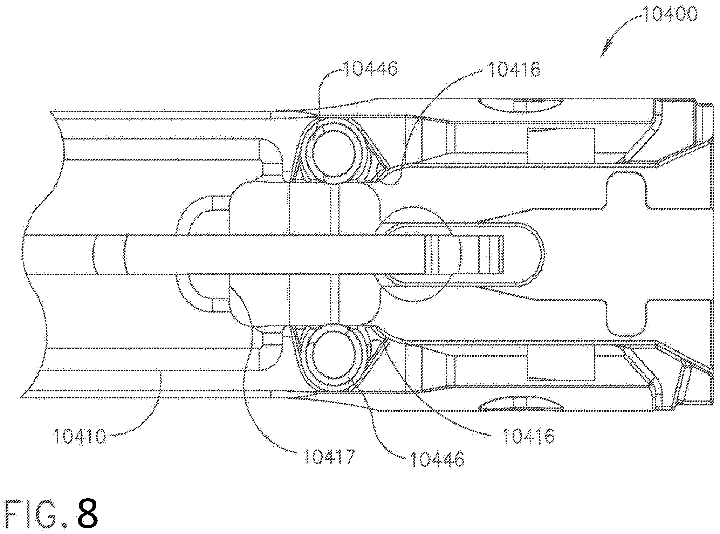

Further to the above, it is desirable for the tissue cutting member 10251 to be in its unfired position at the beginning of the staple firing stroke. If the tissue cutting member 10251 is not in its unfired position at the outset of the staple firing stroke, a missing cartridge/spent cartridge lockout of the stapling instrument 10000 may be accidentally bypassed. Referring to FIG. 8, the lockout of the stapling instrument 10000 comprises a shoulder 10417 defined in the bottom of the staple cartridge jaw 10410. If a proper unspent staple cartridge is seated in the staple cartridge jaw 10410 at the outset of the staple firing stroke, and the tissue cutting member 10251 is in its unfired position at the outset of the staple firing stroke, the tissue cutting member 10251 will be lifted over the lockout shoulder 10417. More specifically, referring to FIG. 13, the nose 10258 of the tissue cutting member 10251 will be supported by a staple driving sled in the staple cartridge such that lockout tabs 10257 of the firing member 10251, and/or any other portion of the firing member 10251, do not contact the lockout shoulder 10417. If, however, a staple cartridge is not seated in the staple cartridge jaw 10410, a staple cartridge is seated the staple cartridge jaw 10410 but has been previously spent, or an incorrect staple cartridge is seated in the staple cartridge jaw 10410, the sled will not support the nose 10258 of the tissue cutting member 10251 and the lockout tabs 10257 will contact the lockout shoulder 10417 at the outset of the staple firing stroke - thereby preventing the staple firing stroke. If the tissue cutting member 10251 is somehow positioned distally with respect to the lockout shoulder 10417 at the outset of the staple firing stroke, however, the advantages provided by the lockout of the surgical instrument 10000 are lost.

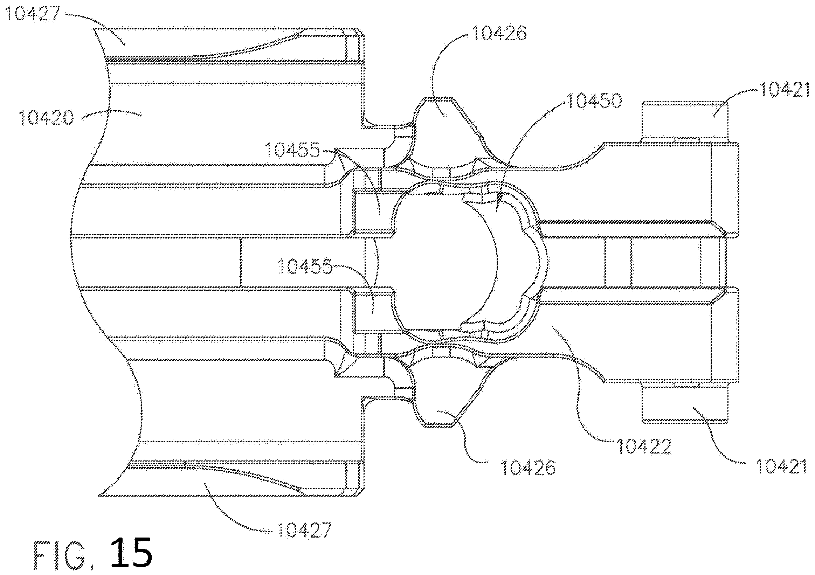

The above being said, referring to FIG. 15, the anvil jaw 10420 comprises shoulders, or stops, 10455 defined thereon which are configured to contact the top cam member 10255 of the tissue cutting member 10251 when the anvil jaw 10420 is moved into its open position. In such instances, the anvil jaw 10420 positions the tissue cutting member 10251 in its unfired position even if the tissue cutting member 10251 has been accidentally moved or positioned too far distally. Such an arrangement is particularly useful after the surgical instrument 10000 has already been used at least once and the staple firing system has been reset, or retracted as, in some instances, the tissue cutting member 10251 may not have been fully returned to its unfired position after the last staple firing stroke. As a result of the above, the possibility of the lockout of the surgical instrument 10000 being accidentally bypassed is reduced. Notably, the shoulders 10455 and the clearance pocket 10450 are positioned proximally with respect to the distal edges of the tissue stops 10427 which assures that the tissue cutting member 10251 is positioned proximally relative to the tissue captured within the end effector such that the tissue is not accidentally incised against the tissue cutting member 10251.

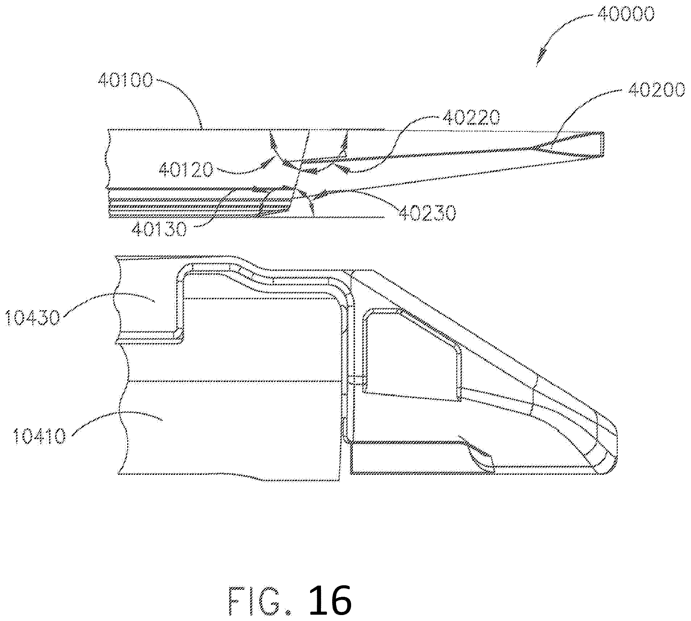

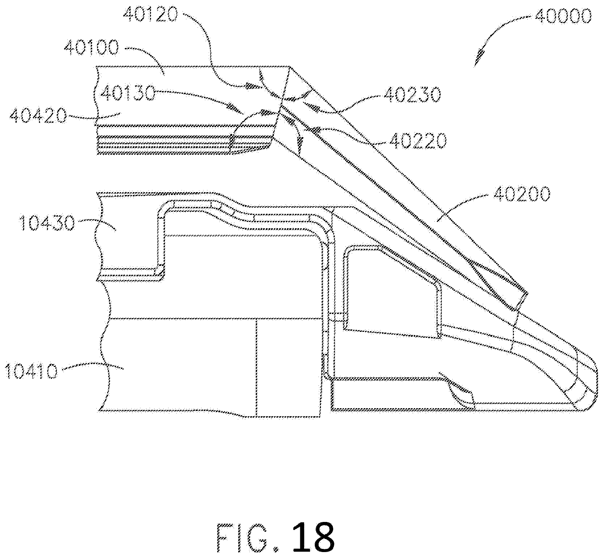

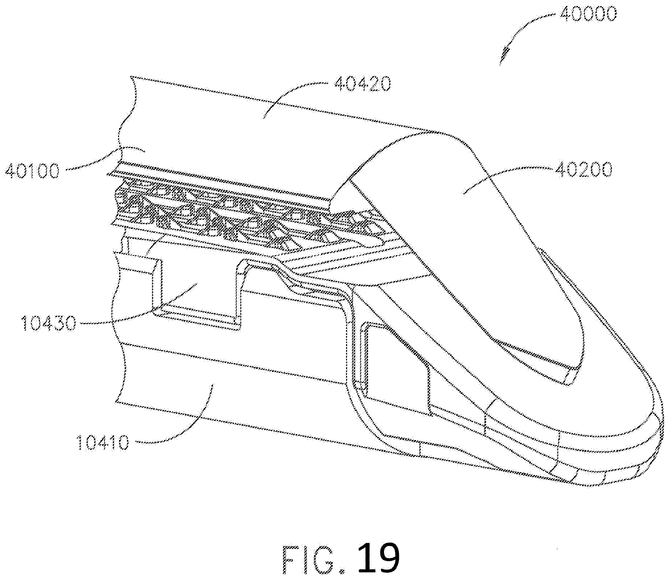

FIGS. 16 -19 depict an end effector 40000 comprising an anvil jaw 40420 and a cartridge jaw 10410. The anvil jaw 40420 comprises a proximal portion 40100 and a distal portion, or tip, 40200 attached to the proximal portion 40100. The distal portion 40200 is rotatable between a first operational orientation (FIG. 16) and a second operational orientation (FIG. 18 and FIG. 19) to provide a clinician with the ability to choose between a straight anvil tip and an angled anvil tip before using the end effector 40000.

The proximal portion 40100 comprises an angled distal end that can be characterized by a first angle 40120 and a second angle 40130. The first angle 40120 is measured with reference to a top plane defined by the top of the proximal portion 40100 while the second angle 40130 is measured with reference to a bottom plane defined by the bottom of the proximal portion 40100. In various instances, the first angle 40120 and the second angle 40130 are supplementary angles. In at least one instance, the first angle 40120 and the second angle 40130 are substantially supplementary. The distal portion 40200 comprises an angled proximal end which is attached to the distal end of the proximal portion 40100. The angled proximal end of the distal portion 40200 can be characterized by a first angle 40220 and a second angle 40230. In various instances, the first angle 40220 and the second angle 40230 are supplementary angles. In at least one instance, the first angle 40220 and the second angle 40230 are substantially supplementary. In various instances, the first angle 40120 and the first angle 40220 are supplementary angles and the second angle 40130 and the second angle 40230 are supplementary angles. This configuration permits the proximal portion 40100 and the distal portion 40200 of the anvil jaw 40420 to have a complimentary, angled attachment plane where a distal face 40110 of the proximal portion 40100 and a proximal face 40210 of the distal portion 40200 abut each other in both the first orientation and the second orientation.

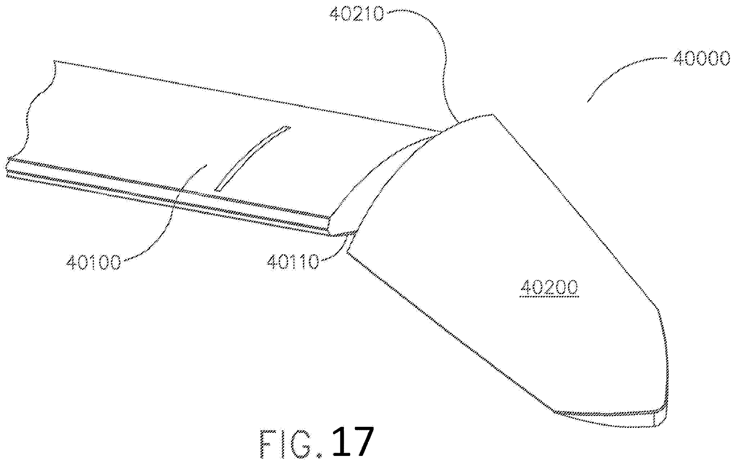

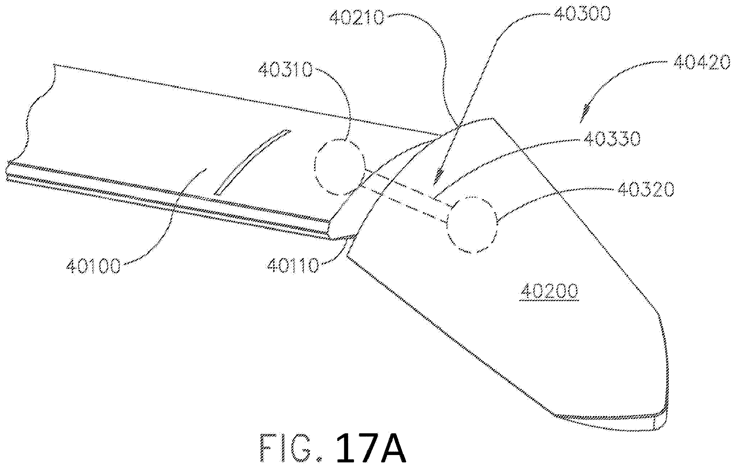

Utilizing an attachment mechanism, referring to FIGS. 17 and 17A, the distal portion 40200 is rotatable relative to the proximal portion 40100 such that the distal portion 40200 can be rotated into different orientations. To move the distal portion 40200 into the second orientation shown in FIG. 18, the distal portion 40200 is rotated 180 degrees from the first orientation show in FIG. 16. This configuration allows a user to change the anvil jaw 40420 between a straight-tipped anvil jaw and an angle-tipped anvil jaw. In the second orientation shown in FIGS. 18 and 19, the first angle 40120 and the second angle 40230 abut each other and, correspondingly, the first angle 40220 and the second angle 40130 abut each other. The angles at the attachment interface in the second orientation (FIG. 18) are not supplementary as they were in the first orientation (FIG. 16).

The attachment mechanism used can be any suitable attachment mechanism. In at least one instance, referring to FIG. 17A, the attachment mechanism comprises a flexible rotatable pin 40300 anchored to the proximal portion 40100 and the distal portion 40200. Such a mechanism allows rotation of the rotatable portion between different orientations while keeping the proximal portion 40100 and the distal portion 40200 attached to each other. One or more spring members and/or detents may be used in conjunction with the pin to hold the portions in either the first operational orientation or the second operational orientation. The attachment mechanism may be embedded in either the proximal portion 40100 and/or the distal portion 40200. The attachment mechanism may comprise a bi-stable compliance mechanism configured to bias the portion 40200 into either orientation to prevent the inadvertent partial rotation of the rotatable distal portion 40200. The attachment mechanism may comprise springloaded detents, a living hinge, sliding members, and/or various other locking members. The attachment mechanisms may also comprise interference and/or friction-fit interfaces between the proximal portion 40100 and the distal portion 40200.

Further to the above, and referring again to FIG. 17A, the flexible pin 40300 comprises a spherical first end 40310 mounted in a chamber defined in the proximal anvil portion 40100, a spherical second end 40320 mounted in a chamber defined in the distal anvil portion 40200, and a flexible connector 40330 connecting the first end 40310 and the second end 40320. The spherical first end 40310 and the spherical second end 40320 can rotate within their respective chambers such that the flexible pin 40300 can rotate relative to the proximal portion 40100 and/or such that the distal portion 40200 can rotate relative to the flexible pin 40300. In either event, such relative rotation permits the rotation of the distal portion 40200 as described above. The length of the flexible connector 40330 is selected such that the flexible connector 40300 is in a resiliently stretched state for every orientation of the distal portion 40200. As a result, the flexible connector 40330 acts to pull the distal portion 40200 against the first anvil portion 40100. Given that the proximal portion 40100 includes the staple forming pockets and the distal portion 40200 does not comprise staple forming pockets, the retention force provided by the pin 40300 does not need to withstand staple forming forces and is sufficient to hold the distal portion 40200 in place while the end effector 40000 is being positioned in the patient. The pin can be spring loaded in the socket such that the spring pulls the head proximally in the chamber thus holding the proximal portion 40100 and the distal portion 40200 together. To rotate the distal portion 40200 between orientations, the distal portion 40200 can be pulled distally to overcome the biasing force, twisted into another orientation, and released so that the spring may pull the distal portion 40200 against the proximal portion 40100. The interface between the distal portion 40200 and the proximal portion may further comprise interlocking features extending therefrom to prevent inadvertent movement relative to each other. For example, teeth may extend from one portion and into corresponding slots defined in the other portion when the distal portion 40200 is in its first and second orientations, but not when the distal portion 40200 is pulled away from the proximal portion 40100.

In at least one instance, the distal portion 40200 comprises two halves, for example, which are assembled around the attachment mechanism. The two halves may utilize an elastomer to hold the halves together around the pin, for example. In at least one instance, a snap-fit mechanism can be used to assemble the two halves together around the attachment mechanism.

In various instances, the proximal portion 40100 and the distal portion 40200 are comprised of one or more materials. For example, the proximal portion 40100 may be comprised of one or more materials and the distal portion 40200 may be comprised of one or more materials. In at least one instance, the distal portion 40200 is comprised of metal toward the attachment interface and is comprised of an over-molded soft tip extending distally from the metal portion. The soft tip may be comprised of rubber and/or plastic, for example. The anvil jaw 40410 may further comprise an intermediate component positioned between the proximal portion 40100 and the distal portion 40200. The intermediate component can house one or more parts of the attachment mechanism. The intermediate component may also provide an atheistically pleasing and/or functional transition piece between the proximal portion 40100 and the distal portion 40200 which may be useful in a scenario where the proximal portion 40100 and the distal portion 40200 comprise more than one material.

In at least one instance, the first portion 40100 and the second portion 40200 comprise edges designed to eliminate any sharp edges presented by rotation of the second portion 40200 relative to the first portion 40100.

As discussed above, the surgical instruments disclosed herein may comprise control systems. Each of the control systems can comprise a circuit board having one or more processors and/or memory devices. Among other things, the control systems are configured to store sensor data, for example. They are also configured to store data which identifies the type of staple cartridge attached to a stapling instrument, for example. More specifically, the type of staple cartridge can be identified when attached to the stapling instrument by the sensors and the sensor data can be stored in the control system. This information can be obtained by the control system to assess whether or not the staple cartridge is suitable for use.

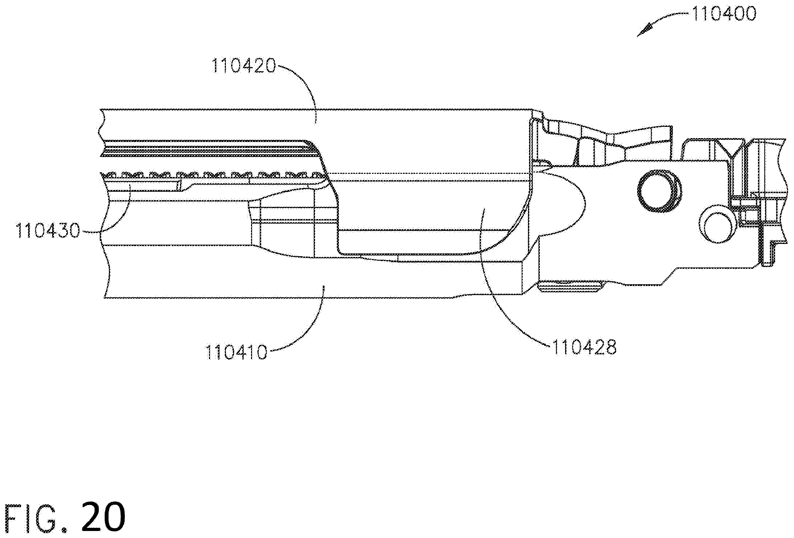

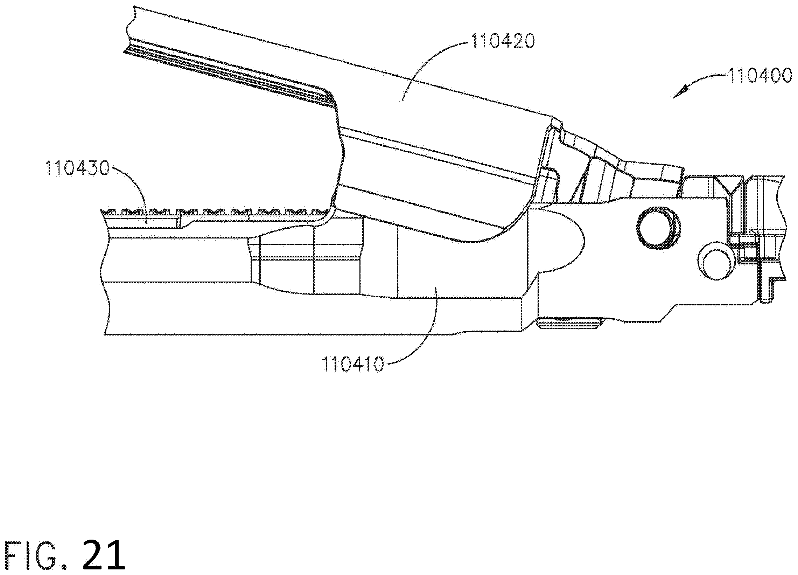

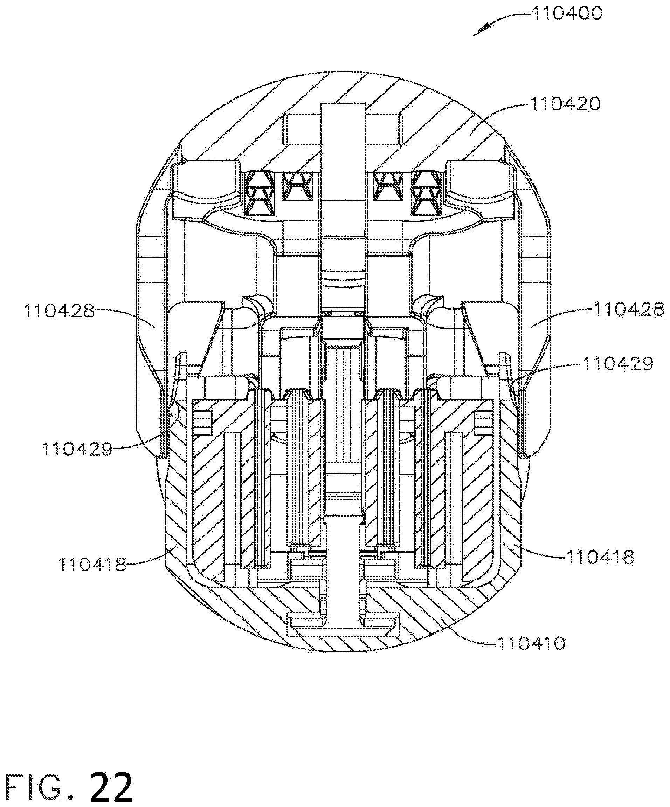

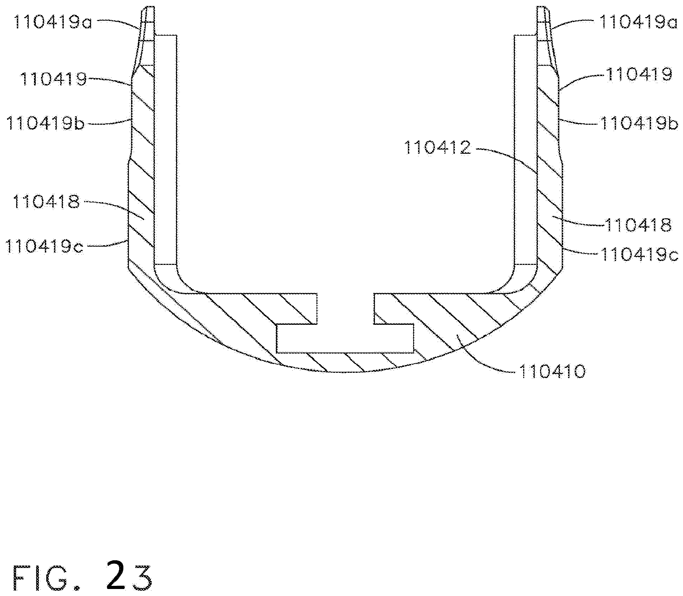

The actuation of the closure drive of the surgical instrument 110000 deactivates the articulation drive system at some point during the closure stroke. As the closure drive reaches the end of its closure stroke, the second jaw 110420 contacts the first jaw 110410 in a manner which indicates to the clinician using the surgical instrument 110000 that the second jaw 110420 is reaching its fully-clamped position. Referring to FIGS. 20-23, the second jaw 110420 is pivotably coupled to the first jaw 110410 and is rotatable between a fully-open position (FIG. 21) and a fully-clamped position (FIG. 20) during the closure stroke. When the second jaw 110420 is in its fully-open position (FIG. 21), the flanges, or tissue stops, 110428 of the second jaw 110420 are not engaged with the first jaw 110410. As the second jaw 110420 is closed, referring to FIG. 22, the tissue stops 110428 come into contact with the outside walls 110418 of the first jaw 110410. The inside surfaces 110429 of the tissue stops are un-angled, or parallel to the closing motion of the second jaw 110420. Referring primarily to FIG. 23, the outside surfaces 110419 of the outside walls 110418 are angled inwardly, or non-parallel to the closing motion of the second jaw 110420. Owing to this arrangement, an interference between the tissue stops 110428 of the second jaw 110420 and the outside walls 110418 of the first jaw 110410 is created during the closing motion of the second jaw 110420 and increases gradually as the second jaw 110420 is moved into its fully-closed position. This increasing interference between the jaws 110410 and 110420 creates an increasing resistance force within the closure drive which is transmitted back through the closure tube 110240 into the closure trigger 10140. The clinician pulling the closure trigger 10140 can feel the increasing resistance force being transmitted through the closure trigger 10140 and understand that the second jaw 10420 is reaching its fully-closed position.

Referring primarily to FIG. 23, each outside surface 110419 comprises a top angled surface 110419a, a second angled surface 110419b, and a final angled surface 110419c, for example. The first jaw 110410 comprises a channel 110412, which is configured to receive a staple cartridge therein, which comprises a top width defined between the top angled surfaces 110419a. The top width of the channel 110412 is narrower than an intermediate width defined between the intermediate angled surfaces 110419b which is narrower than a final width defined between the final angled surfaces 110419c. In addition to providing a tactile feedback to the clinician, the above-described arrangement maintains a proper lateral alignment between the first jaw 110410 and the second jaw 110420.

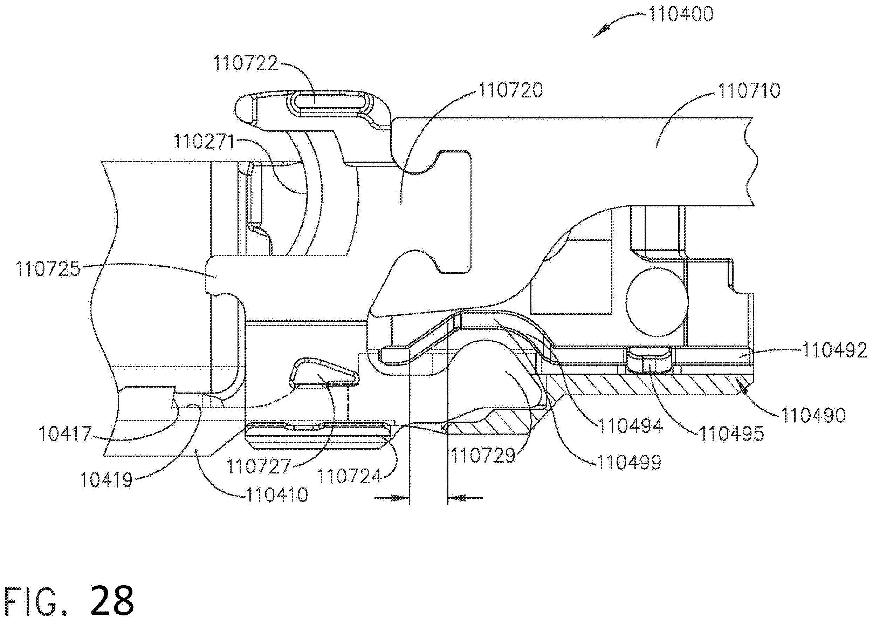

Once the end effector 110400 has been sufficiently closed the staple firing drive of the surgical instrument 110000 can be actuated to fire the staples contained in the staple cartridge seated in the end effector 110400 during a staple firing stroke. Referring to FIG. 28, the staple firing drive comprises a firing member, or bar, 110710 which is advanced distally by the electric motor of the staple firing drive in response to an actuation of the firing trigger. The staple firing drive further comprises a coupling element 110720 attached to the distal end of the firing bar 110710. In at least one embodiment, the interface between the firing bar 110710 and the coupling element 110720 comprises a dovetail arrangement, for example. The coupling element 110720 is moveable between a proximal unfired position, illustrated in FIG. 28, and a distal fired position during a staple firing stroke. The coupling element 110720 comprises a cam 110724 configured to engage the first jaw 110410 and a cam 110722 configured to engage the second jaw 110420 during the staple firing stroke and hold the second jaw 110420. The cams 110722 and 110724 co-operate to hold the second jaw 110420 in position relative to the first jaw 110410 during the staple firing stroke, although embodiments are envisioned without the cams 110722 and 110724. The coupling element 110720 further comprises a tissue cutting edge 110271 configured to transect the tissue captured between the first jaw 110410 and the second jaw 110420 during the staple firing stroke.

Further to the above, the surgical instrument 110000 comprises a staple firing lockout to prevent the staple firing stroke when a staple cartridge is missing from the first jaw 110410 and/or when the staple cartridge seated in the first jaw 110410 has already been at least partially fired. To this end, the coupling element 110720 further comprises a proximally-extending tail 110729 which is biased downwardly, i.e., toward the bottom of the first jaw 110410, at the beginning of the staple firing stroke by a firing lockout spring 110490 mounted in the shaft 110200. Prior to the beginning of the firing stroke, referring to FIG. 28, the lockout spring 110490 is in an initially-unloaded state. In other words, the lockout spring 110490 does not apply any force to the proximally-extending tail 110729 of the coupling element 110720 when the coupling element 110720 is in its unfired position. Once the coupling element 110720 is advanced distally at the beginning of the staple firing stroke, the tail 110729 comes into contact with the lockout spring 110490 such that the lockout spring 110490 applies a downward force to the coupling element 110720. As discussed below, the downward force applied to the coupling element 110720 can, in various instances, lockout the staple firing drive and prevent the coupling element 110720 from being moved distally through the staple firing stroke. Such a configuration can avoid a continuous, and unnecessary, stress experienced by a firing assembly over time by only applying a lockout force to the firing assembly during the beginning of a firing stroke instead of applying a lockout force for an indefinite amount of time prior to the beginning of the firing stroke.

If an unfired staple cartridge is not seated in the first jaw 110410 at the beginning of the staple firing stroke, the firing lockout spring 110490 will push the coupling element 110720 downwardly such that a laterally-extending lock shoulder 110727 extending from the coupling element 110720 enters into a lock recess 10419 defined in the first jaw 10410 and contacts a lock shoulder 10417 at the distal end of the lock recess 10419 which blocks the distal advancement of the staple firing drive to prevent the staple firing stroke. If an unfired staple cartridge is seated in the first jaw 110410 at the beginning of the staple firing stroke, a distal end 110725 of the coupling element 110720 is supported by a sled in the staple cartridge which prevents the coupling element 110720 from being pushed into the lock recess 110419 by the firing lockout spring 110490 and, as a result, the coupling element 110720 can be advanced distally to perform the staple firing stroke. As the coupling element 110720 is being advanced distally through the staple firing stroke, the tail 110729 is no longer in contact with the firing lockout spring 110490 and, thus, the downward biasing force is not applied to the staple firing drive through the staple firing stroke. When the coupling element 117020 is retracted proximally after the staple firing stroke, the tail 110729 re-engages the lockout spring 110490 and deflects the lockout spring 110490 upwardly such that the tail 110729 can slide under the lockout spring 110490 back into its unfired position.

Further to the above, the firing lockout spring 110490 comprises a proximal portion 110492 mounted to the shaft 110200 and a distal end 110494 which is free to move relative to the proximal portion 110492. The distal end 110494 comprises an arcuate portion 110499 which extends over the proximal tail 110729 which is contacted by the proximal tail 110729 when the staple firing drive is actuated. The firing lockout spring 110490 further comprises lateral supports 110495 extending therefrom which supports the distal end 110494 over the proximal tail 110729. The lateral supports 110495 are positioned within recesses 110415 defined in the first jaw 110410 which hold the lateral supports 110495 in position. As a result of this arrangement, the firing lockout spring 110490 is prevented from bottoming out on the first jaw 110410 and shortening the effective length of the firing lockout spring 110490. Moreover, as a result of this arrangement, the firing lockout spring 110490 is able to flex and/or move upwardly to permit the coupling member 110720 to pass thereby without yielding or permanently deforming the firing lockout spring 110490. When the coupling member 110720 is returned to its proximal unfired position after the staple firing stroke, the firing lockout spring 110490 is moved upwardly by the coupling member 110720 to permit the coupling member tail 110729 to move thereunder.

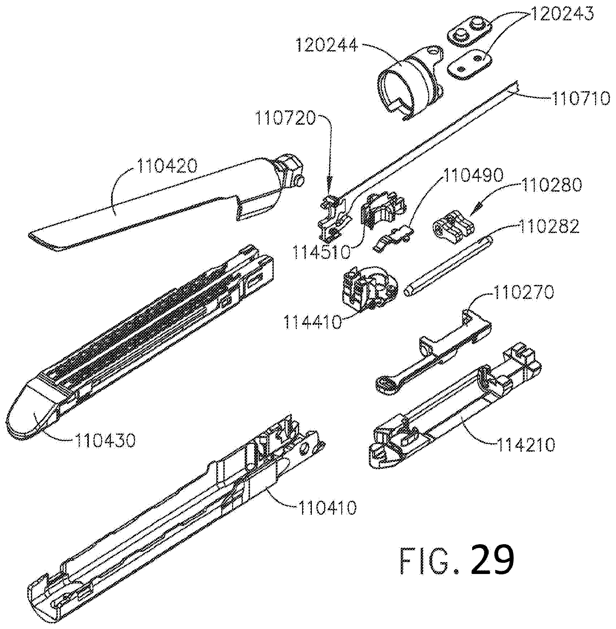

The end effector 110400 comprises a cartridge jaw 110410 and an anvil jaw 110420 rotatable relative to the cartridge jaw 110410 between an open position and a closed position. The surgical instrument 120000 further comprises a closure drive that is actuated by a closure trigger 10140 to move the anvil jaw 110420 into its closed position. More specifically, the closure trigger 10140 comprises a crank that pushes a closure tube 120240 of the shaft 120200 distally which contacts the anvil jaw 110420 and pushes the anvil jaw 110420 into its closed position. In alternative embodiments, the actuation of the closure trigger 10140 can operate an electric motor which drives the closure tube 120240. That said, embodiments having a mechanical coupling between the closure trigger 10140 and the closure tube 120240 provides the clinician with a feel of the clamping force being applied to the patient tissue while, on the other hand, a motor-driven system can assist the clinician with overcoming high clamping loads. In either event, the closure tube 120240 extends across the articulation joint 120500 and comprises a joint arrangement which accommodates the articulation of the end effector 110400 about the articulation joint 120500. Referring to FIG. 29 , the closure tube 120240 includes a proximal portion 120242 engaged with a closure carriage slideable within the handle 120100, a distal portion 120244 positioned on a distal side of the articulation joint 120500, and links 120243 rotatably connecting the distal portion 120244 to the proximal portion 120242. The links 120243 extend across and are generally aligned with the articulation joint 120500 which permit the closure tube 120240 to slide distally regardless of the articulated orientation of the end effector 110400.

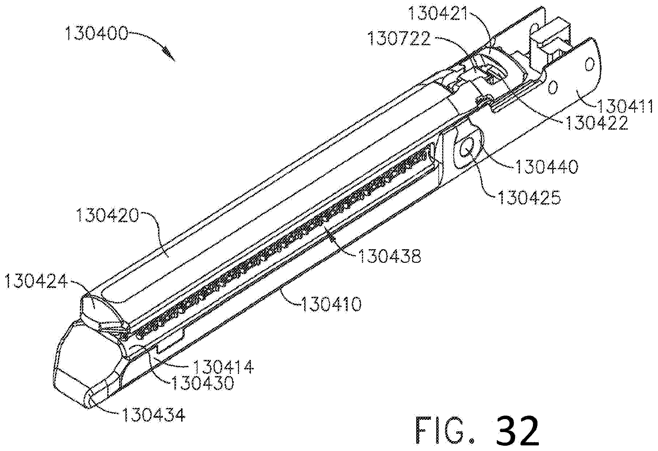

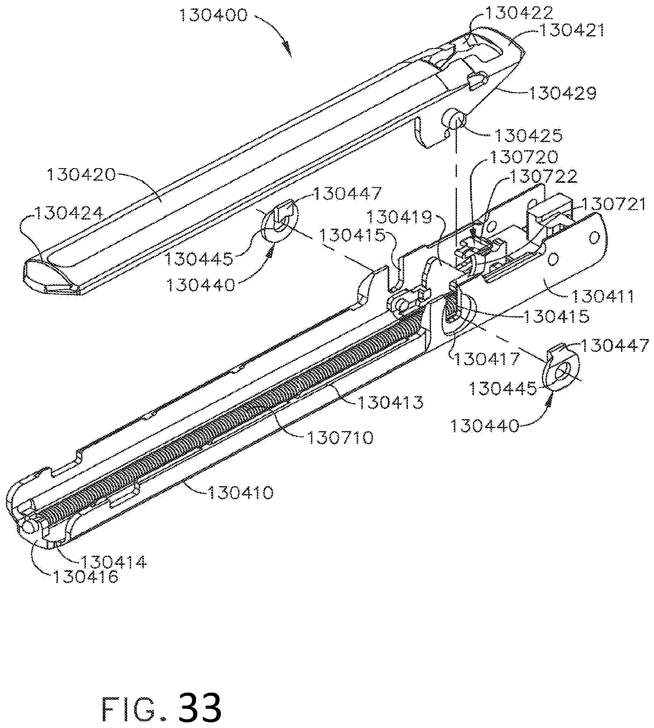

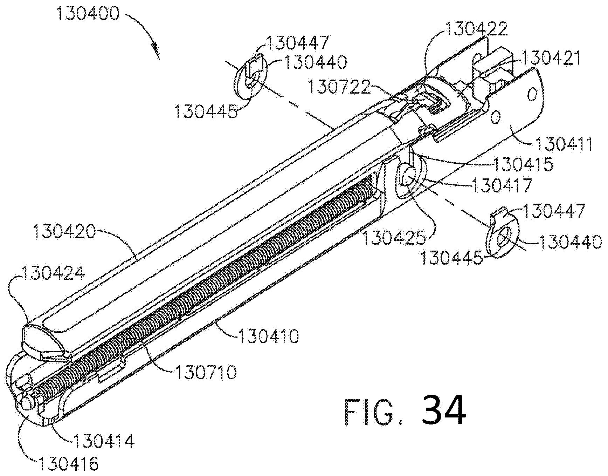

An end effector 130400 is illustrated in FIGS. 32-35. The end effector 130400 comprises a cartridge jaw 130410 and an anvil jaw 130420 rotatably coupled to the cartridge jaw 130410. The cartridge jaw 130410 comprises a proximal end 130411, a distal end 130414, and a channel 130413 extending between the proximal end 130411 and the distal end 130414. The channel 130413 is configured to closely receive a staple cartridge 130430 therein such that there is little, if any, relative movement between the staple cartridge 130430 and the channel 130413. The staple cartridge 130430 and the channel 130413 comprise co-operating snap-fit and/or press-fit features which releasably hold the staple cartridge 130430 in the cartridge jaw 130410. The staple cartridge 130430 comprises a cartridge body including staple cavities 130438 defined therein and staples removably stored in the staple cavities 130438. The staple cartridge 130430 further comprises staple drivers configured to support the staples and a sled movable from a proximal position to a distal position to drive the staple drivers and staples toward the anvil jaw 130420 during a staple firing stroke. When the staple cartridge 130430 is seated in the channel 130413, the staple cartridge 130430 is positioned over a rotatable drive screw 130710 extending through the cartridge jaw 130410 such that the sled is positioned in front of a tissue cutting knife 130720 threadably engaged with the drive screw 130710. The rotatable drive screw 130710 comprises a proximal end operably coupled to an electric motor in the handle of the surgical instrument and a distal end rotatably supported by a bearing 130416 defined in the distal end 130414 of the cartridge jaw 130410. When the drive screw 130710 is rotated in a first direction, the drive screw 130710 drives the tissue cutting knife 130420 distally from an unfired position to push the sled through the staple firing stroke. When the drive screw 130710 is rotated in a second, or opposite, direction, the drive screw 130710 retracts the tissue cutting knife 130720 back into its unfired position. Notably, the sled is not retracted proximally with the tissue cutting knife 130720. As a result, in various embodiments, the sled can be used as part of a missing cartridge/spent cartridge lockout which prevents a spent staple cartridge from being fired after it has been previously fired.

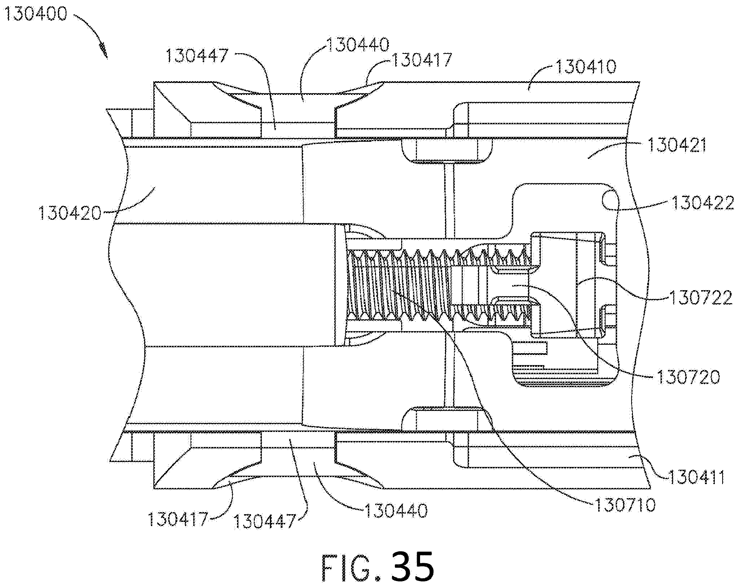

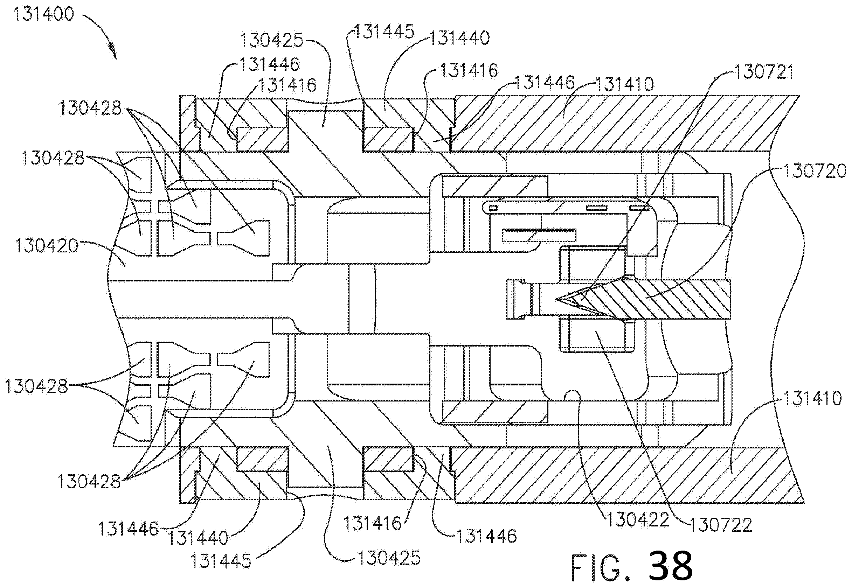

Further to the above, the anvil jaw 130420 comprises a proximal end 130421 rotatably coupled to the cartridge jaw 130410 about a pivot, discussed further below, and a distal end 130424 movable toward and away from a distal end 130434 of the staple cartridge 130430. The anvil jaw 130420 further comprises staple forming pockets 130428 (FIG. 38) defined therein which are registered with the staple cavities 130438, and the staples positioned in the staple cavities 130438, of the staple cartridge 130430 when the anvil jaw 130420 is in a clamped position. Moreover, the anvil jaw 130420 further comprises an opening 130422 defined therein which is configured to receive and provide clearance for a top flange 130722 of the tissue cutting knife 130720 when the anvil jaw 130420 is in its closed position. Further to the above, the tissue cutting knife 130720 is movable distally from its proximal unfired position (FIG. 35) to perform the staple firing stroke and cut patient tissue with a knife edge 130271 defined thereon. When the tissue cutting knife 130720 is moved distally, the top flange 130722 is no longer aligned with the opening 130422 and, instead, the top flange 130722 enters into an internal longitudinal slot defined in the anvil jaw 130420. The flange 130722 is configured to hold the anvil jaw 130420 in position during the staple firing stroke in cooperation with a bottom flange extending from the tissue cutting knife 130720 that is engaged with the cartridge jaw 130410. In such instances, the anvil jaw 130420 cannot be re-opened unless the tissue cutting knife 130720 is retracted proximally out of engagement with the anvil jaw 130420.

Further to the above, the anvil jaw 130420 comprises a first lateral side, a second lateral side, and a trunnion 130425 extending from each of the first and second lateral sides. The cartridge jaw 130410 comprises a first lateral sidewall closely aligned with the first lateral side of the anvil jaw 130420, a second lateral sidewall closely aligned with the second lateral side of the anvil jaw 130420, and an alignment slot 130415 defined in each of the first and second lateral sidewalls which closely receive a trunnion 130425 therein. The close lateral alignment between the cartridge jaw 130410 and the anvil jaw 130420 prevents the anvil jaw 130420 from sliding laterally relative to the cartridge jaw 130410. In various instances, there is a line-to-line lateral fit between the anvil jaw 130420 and the cartridge jaw 130410. Similarly, in various instances, there is a line-to-line fit between the trunnions 130425 and the sidewalls of the alignment slots 130415 which prevents the anvil jaw 130420 from sliding longitudinally relative to the cartridge jaw 130410 but permits the anvil jaw 130420 to rotate relative to the cartridge jaw 130410. That said, the rotation, or opening, of the anvil jaw 130420 is constrained when stop surfaces 130429 defined on the proximal end 130421 of the anvil jaw 130420 contact stops 130419 defined in the cartridge jaw 130410.

When the anvil jaw 130420 is assembled to the cartridge jaw 130410, further to the above, the trunnions 130425 are aligned with the open ends of the alignment slots 130415 defined in the cartridge jaw 130410 and then pushed into contact with the bottom ends of the alignment slots 130415. To retain the trunnions 130425 in this position, retaining clips 130440 are assembled to the trunnions 130425 and then welded to the cartridge jaw 130410. Each retaining clip 130440 comprises an aperture 130445 defined therein which is slid over and closely receives a trunnion 130425 therein. The trunnion 130425 is cylindrical, or at least substantially cylindrical, and the aperture 130445 is circular, or at least substantially circular. The trunnion 130425 comprises an outer diameter which is equal to, or slightly smaller than, the diameter of the aperture 130445 such that the trunnion 130425 rotates within, but does not translate relative to, the retaining clip 130440. Each retaining clip 130440 further comprises a circular, or an at least substantially circular, body and an alignment arm 130447 extending therefrom. The circular body of each retaining clip 130440 is closely received within a circular, or an at least substantially circular, recess 130417 defined in an outwardly-facing surface of the first and second lateral sidewalls of the cartridge jaw 130410. The alignment arm 130447 of each retaining clip 130440 comprises a rectangular member, or an at least substantially rectangular member, closely received within an alignment slot 130415. In various instances, each alignment slot 130415 comprises parallel, or at least substantially parallel, sidewalls extending from the open end of the alignment slot 130415 to the closed end. In at least one embodiment, the sidewalls of each alignment slot 130415 comprise tapered sidewalls extending from a larger open end to a smaller closed end. In either event, the alignment arms 130447 of the retaining clips 130440 interact with the sidewalls of the alignment slots 130415 which prevents the anvil jaw 130420 from sliding relative to the cartridge jaw 130410. In various instances, the retaining clips 130440 are welded to the cartridge jaw 130410 along the perimeter, or interface, between the retaining clips 130440 and the cartridge jaw 130410 to hold the retaining clips 130440 in position.

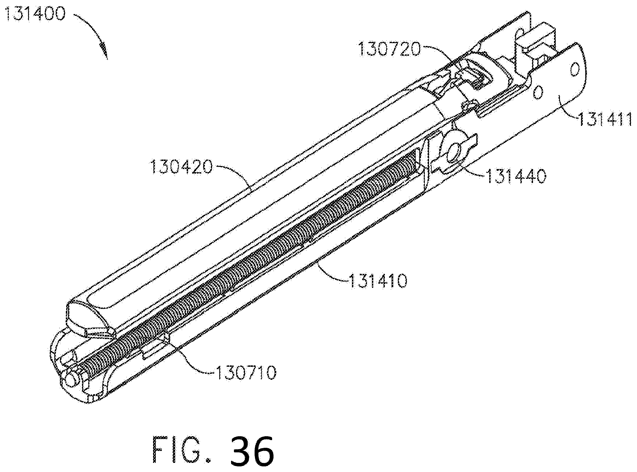

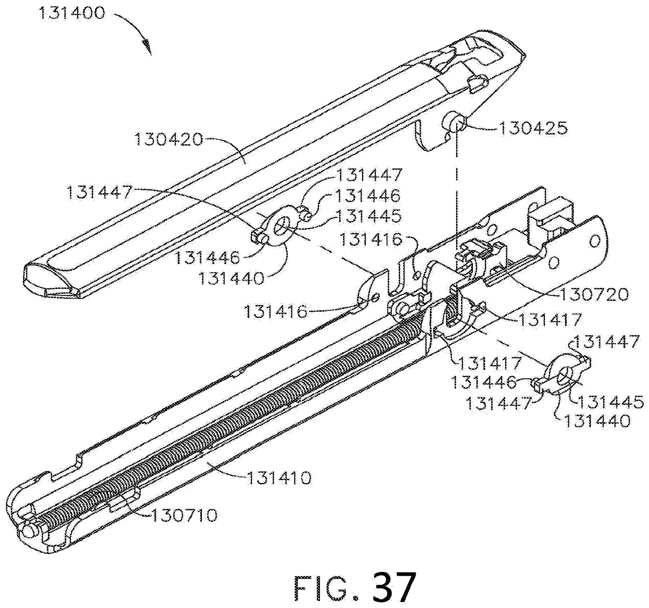

An end effector 131400 is illustrated in FIGS. 36-38 and is similar to the end effector 130400 in many respects, most of which will not be repeated herein for the sake of brevity. The end effector 131400 comprises a cartridge jaw 131410 and retaining clips 131440 which hold the anvil jaw 130420 to the cartridge jaw 131410. Similar to the above, the retaining clips 131440 prevent the anvil jaw 130420 from translating relative to the cartridge jaw 131410 but permit the anvil jaw 130420 to rotate relative to the cartridge jaw 131410 between open and closed positions. Each retaining clip 131440 comprises a circular body closely received within a circular recess defined in the cartridge jaw 131410 and alignment flanges 131447 extending therefrom which are closely received in corresponding recesses 131417 defined in the cartridge jaw 131410. The alignment flanges 131447 and the recesses 131417 are sized and configured to prevent the retaining clips 131440 from rotating relative to the cartridge jaw 131410. Moreover, the alignment flanges 131447 of each retaining clip 131400 extends in opposite directions and co-operate to resist the lateral, or sideways, rotation of the anvil jaw 130420 relative to the cartridge jaw 131410. Each retaining clip 131440 provides two points of control via the alignment flanges 131447 resulting in a four-point control of the anvil jaw 130420. Additionally, each alignment flange 131447 comprises a control peg, or pin, 131446 extending therefrom which is closely received in an aperture 131416 defined in the cartridge jaw 131410 which assists in stabilizing the retaining clip 131440 in position. Similar to the above, the retaining clips 131440 are welded and/or otherwise suitably affixed to the cartridge jaw 131410 to hold the anvil jaw 130420 in position.

As discussed above, the retaining clips 130440 and 131440 hold the anvil jaw 130420 in alignment with the cartridge jaw 130410. More specifically, the retaining clips 130440 and 131440 hold the staple forming pockets 130428 (FIG. 38) in registration with the staples stored in the staple cartridge 131430 such that the staples are properly deformed during the staple firing stroke. That said, the method used to assemble the end effectors 130400 and 131400 can have a large impact on reliably aligning the staple forming pockets 130428 with the staples. In various instances, a jig, or fixture, is used to assemble the end effector 130400 and/or 131400. In at least one instance, with reference to the end effector 130400, the cartridge jaw 130410 is seated in a first portion of the jig and the anvil jaw 130420 is positioned on top of the cartridge jaw 130410 such that the trunnions 130425 are positioned in the alignment slots 130415, as discussed above. At such point, a second portion of the jig is moved toward the first portion of the jig to cam, or nudge, the anvil jaw 130420 into its proper position relative to the cartridge jaw 130410 and hold the anvil jaw 130420 in its proper positon. In at least one embodiment, the jig comprises a lock which holds the second portion of the jig in position relative to the first portion. In any event, the first and second portions of the jig comprise control surfaces which control the position of the cartridge jaw 130410 and the anvil jaw 130420 within the jig. Once the jig is closed, the retaining clips 130440 are assembled to the trunnions 130425 and welded, or otherwise affixed, to the cartridge jaw 130410. The jig is opened to release the end effector 130400. At such point, the anvil jaw 130420 is properly secured in position such that the staple forming pockets 130428 will be aligned with the staples of a staple cartridge 130430 that is seated in the cartridge jaw 130410.

Further to the above, a staple cartridge is insertable into, and seatable within, a cartridge jaw of a stapling instrument. In various embodiments, the cartridge jaw comprises a stop, or datum, against which the staple cartridge can contact to make sure that the staple cartridge is seated in the correct position in the cartridge jaw. The stop is configured to prevent the staple cartridge from being inserted too deeply, i.e., proximally, into the end effector. Without such a stop, it is possible - in some instances and in some embodiments - for the staple cartridge to be inserted too deeply into the end effector such that the staple cartridge accidentally contacts the tissue cutting knife, for instance. In some such instances, the sled positioned in the staple cartridge may contact the tissue cutting knife before the staple cartridge is fully seated and, as a result, the sled may be accidentally, and prematurely, pushed distally within the staple cartridge from its unfired position. A sled in such a disturbed position would not be able to defeat or deactivate a staple firing lockout that keys off of the sled being in its unfired position at the outset of the staple firing stroke of the tissue cutting knife.

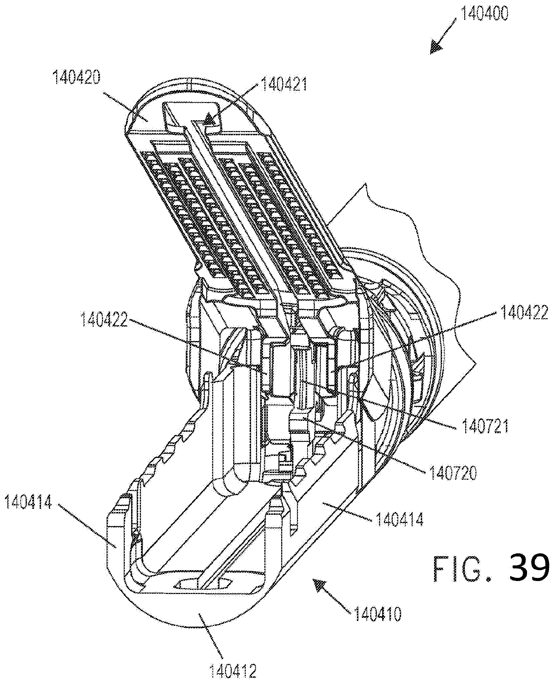

In addition to or in lieu of a cartridge stop in a cartridge jaw as discussed above, the anvil jaw of an end effector can comprise a cartridge stop configured to prevent a staple cartridge from being inserted too deeply, i.e., proximally, within the end effector. Referring to FIGS. 39-41, an end effector 140400 of a surgical stapling instrument comprises a cartridge jaw 140410 and an anvil jaw 140420. The anvil jaw 140420 is rotatable relative to the cartridge jaw 140410; however, in various other embodiments, the cartridge jaw 140410 is rotatable relative to the anvil jaw 140420. In either event, the cartridge jaw 140410 is configured to receive a staple cartridge 140430 therein. The cartridge jaw 140410 defines a channel including a bottom wall 140412 and lateral sidewalls 140414 extending upwardly from the bottom wall 140412. When the staple cartridge 140430 is inserted into the channel, a proximal end 140432 of the staple cartridge 140430 is inserted between the cartridge jaw 140410 and the anvil jaw 140420 by a clinician and moved proximally into the end effector 140400. In many instances, the clinician is able to visually align the staple cartridge 140430 between the lateral sidewalls 140414 and visually determine the proper depth, i.e., proximal-to-distal position, of the staple cartridge 140430 along a longitudinal axis LA before seating the staple cartridge 140430 into the cartridge jaw 140410. The cartridge jaw 140410 and/or the staple cartridge 140430 comprise snap-fit and/or press-fit features which releasably secure the staple cartridge 140430 in the cartridge jaw 140410 when the staple cartridge 140430 is pushed into, i.e., seated within, the cartridge jaw 140410. In some instances, however, the clinician may mistakenly attempt to insert the staple cartridge 140430 too deeply, i.e., proximally, into the end effector 140400 when attempting to seat the staple cartridge 140430 within the cartridge jaw 140410. As such, the anvil jaw 140420 comprises cartridge stops 140422 which are configured to prevent the staple cartridge 140430 from contacting a firing bar 140710 of the stapling instrument when the staple cartridge 140430 is inserted into the end effector 140400.

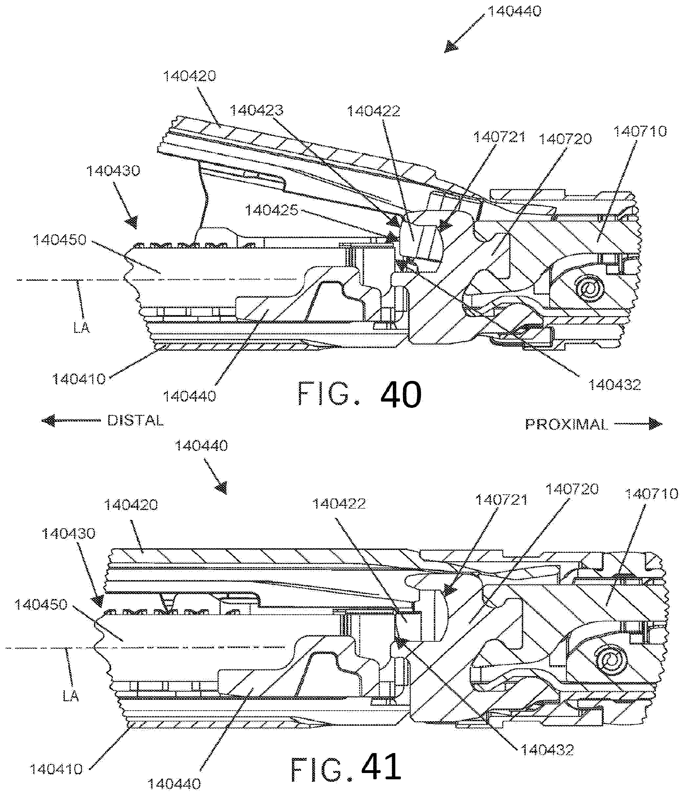

Further to the above, the firing bar 140710 comprises a distal knife head 140720 including a tissue knife edge 140721 configured to cut patient tissue during a staple firing stroke, a first cam configured to engage the anvil jaw 140420 during the staple firing stroke, and a second cam configured to engage the cartridge jaw 140410 during the staple firing stroke. When the anvil jaw 140420 is in its open configuration and the firing bar 140710 has not been advanced through the staple firing stroke, referring to FIG. 40, the cartridge stops 140422 extend distally with respect to the firing bar 140710 such that the staple cartridge 140430 cannot contact the firing bar 140710 when the staple cartridge 140430 is being loaded proximally into the end effector 140400. As a result, a sled 140440 positioned in a cartridge body 140450 of the staple cartridge 140430 isn't accidentally pushed forward out of its proximal unfired position (FIGS. 40 and 41) by accidentally contacting the firing bar 140710 prior to the staple firing stroke.

Once the staple cartridge 140430 has been properly seated in the cartridge jaw 140410, further to the above, the end effector 140400 can be moved into its clamped configuration (FIG. 41) such that the end effector 140400 can be inserted into a patient and/or clamped onto patient tissue. When the staple firing stroke is initiated, the firing bar 140710 is advanced distally past the cartridge stops 140422 to fire the staples stored in the cartridge body 140450. Stated another way, the cartridge stops 140422 are positioned and arranged such that they do not interfere with the firing bar 140710 during the staple firing stroke and/or when the firing bar 140710 is retracted back into its unfired position. Each cartridge stop 140422 comprises a tab, shoulder, wall, and/or flange, for example, extending downwardly from the anvil jaw 140420, i.e., toward the cartridge jaw 140410, such that the cartridge stops 140422 extend downwardly on the lateral sides of the firing bar 140710. That being said, the cartridge stops 140422 can comprise any suitable configuration and, in at least one embodiment, an anvil jaw 140420 may have only one cartridge stop 140422.

As a result of the above, the possibility of the sled 140440 being accidentally displaced distally out of its proximal unfired position prior to the staple firing stroke is reduced. When the sled 140440 is in its proximal unfired position when the staple firing stroke is initiated, further to the above, the sled 140440 can defeat or overcome a firing stroke lockout at the beginning of the staple firing stroke which permits the staple firing stroke to be performed. When the sled 140440 is not in its proximal unfired position when the staple firing stroke is initiated, the sled 140440 does not defeat or overcome the firing stroke lockout at the beginning of the staple firing stroke which prevents the staple firing stroke from being performed.

Further to the above, referring again to FIGS. 40 and 41, the proximal end 140432 of the staple cartridge 140430 is positioned close to, but distally with respect to, the cartridge stops 140422 when the staple cartridge 140430 is seated in the cartridge jaw 140410. The cartridge stops 140422 extend behind the proximal end 140432 of the staple cartridge 140430 when the end effector 140400 is in its open configuration (FIG. 40) and in its clamped configuration (FIG. 41) such that there is clearance between the cartridge 140430 and the cartridge stops 140422 so that the end effector 140400 can be opened and closed.

Notably, further to the above, the distal knife head 140720 moves within a longitudinal slot 140421 defined in the anvil jaw 140420 between the cartridge stops 140422 during the staple firing stroke. The cartridge stops 140422 extend behind the staple cartridge 140430 on opposite sides of the longitudinal slot 140421. Also notably, the distal knife head 140720 moves within a longitudinal slot defined in the staple cartridge 140430 during the staple firing stroke. The longitudinal slot defined in the staple cartridge 140430 divides the proximal end 140432 of the staple cartridge 140430 into a first lateral side and a second lateral side and is aligned with, or substantially aligned with, the longitudinal slot 140421 in the anvil jaw 140420. As a result of this arrangement, one of the cartridge stops 140422 extends behind the first lateral side of the staple cartridge 140430 and the other cartridge stop 140422 extends behind the second lateral side of the staple cartridge 140430.

Further to the above, each cartridge stop 140422 comprises a distal edge 140423 which faces the staple cartridge 140430. Each distal edge 140423 comprises one or more surfaces which are configured to block the proximal movement of the staple cartridge 140430 without contacting the sled 140440. For instance, referring to FIG. 40, the distal edge 140423 of each cartridge stop 140422 comprises a flat, or an at least substantially flat, surface 140425 which faces the staple cartridge 140430 when the end effector 140400 is in its open configuration and the staple cartridge 140430 is inserted into the end effector 140400. If the staple cartridge 140430 is inserted too deeply into the end effector 140400, the cartridge body 140450 of the staple cartridge 140430 contacts the cartridge stops 140422 instead of the sled 140440 contacting the cartridge stops 140422. If the sled 140440 were to contact the cartridge stops 140422, the staple cartridge 140430 would become spoiled and unuseable, as described above, and would need to be replaced. As a result of the arrangement of the end effector 140400, the staple cartridge 140430 may not be accidentally spoiled before it is fired which can reduce time during the surgical procedure and reduce the frustration of the clinician.

The surgical instrument systems described herein are motivated by an electric motor; however, the surgical instrument systems described herein can be motivated in any suitable manner. In certain instances, the motors disclosed herein may comprise a portion or portions of a robotically controlled system. U.S. Patent Application Serial No. 13/118,241, entitled SURGICAL STAPLING INSTRUMENTS WITH ROTATABLE STAPLE DEPLOYMENT ARRANGEMENTS, now U.S. Patent No. 9,072,535, for example, discloses several examples of a robotic surgical instrument system in greater detail.

The surgical instrument systems described herein have been described in connection with the deployment and deformation of staples; however, the embodiments described herein are not so limited. Various embodiments are envisioned which deploy fasteners other than staples, such as clamps or tacks, for example. Moreover, various embodiments are envisioned which utilize any suitable means for sealing tissue. For instance, an end effector in accordance with various embodiments can comprise electrodes configured to heat and seal the tissue. Also, for instance, an end effector in accordance with certain embodiments can apply vibrational energy to seal the tissue.

Although various devices have been described herein in connection with certain embodiments, modifications and variations to those embodiments may be implemented. Particular features, structures, or characteristics may be combined in any suitable manner in one or more embodiments. Thus, the particular features, structures, or characteristics illustrated or described in connection with one embodiment may be combined in whole or in part, with the features, structures or characteristics of one ore more other embodiments without limitation. Also, where materials are disclosed for certain components, other materials may be used. Furthermore, according to various embodiments, a single component may be replaced by multiple components, and multiple components may be replaced by a single component, to perform a given function or functions. The foregoing description and following claims are intended to cover all such modification and variations.

The devices disclosed herein can be designed to be disposed of after a single use, or they can be designed to be used multiple times. In either case, however, a device can be reconditioned for reuse after at least one use. Reconditioning can include any combination of the steps including, but not limited to, the disassembly of the device, followed by cleaning or replacement of particular pieces of the device, and subsequent reassembly of the device. In particular, a reconditioning facility and/or surgical team can disassemble a device and, after cleaning and/or replacing particular parts of the device, the device can be reassembled for subsequent use. Those skilled in the art will appreciate that reconditioning of a device can utilize a variety of techniques for disassembly, cleaning/replacement, and reassembly. Use of such techniques, and the resulting reconditioned device, are all within the scope of the present application.

The devices disclosed herein may be processed before surgery. First, a new or used instrument may be obtained and, when necessary, cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, and/or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta radiation, gamma radiation, ethylene oxide, plasma peroxide, and/or steam.

While this invention has been described as having exemplary designs, the present invention may be further modified within the scope of the claims.

## Claims

1. A surgical stapling assembly (151000, 152000, 152500), comprising:
a staple cartridge (10430, 130430, 140430) comprising a longitudinal slot and a plurality of staples removably stored within said staple cartridge,
a first jaw (10420, 110420, 130420, 140420) comprising coupling pins (130425) extending laterally outward from a proximal end (130421) of said first jaw (10420, 110420, 130420, 140420);
a second jaw (10410, 110410, 130410, 140410), wherein one of said first jaw and said second jaw is movable relative to the other of said first jaw and said second jaw, and wherein said second jaw comprises:
recesses (130417, 131417) defined in said second jaw; and
slots (130415) defined in said second jaw that are configured to receive said coupling pins (130425) to pivotably couple said first jaw and said second jaw; and
coupling members (110720, 130440, 131440) positioned within said recesses, wherein said coupling pins are received within pivot apertures (10415) defined in said coupling members such that said coupling members constrain translation between said first jaw and said second jaw such that the only motion between said first jaw and said second jaw comprises rotation about a pivot axis defined by said coupling pins and said pivot apertures; and **characterized in that**
the slots defined in the second jaw extend partially through the recesses; and the coupling members are welded to the second jaw.

2. The surgical stapling assembly of Claim 1, wherein each said coupling member comprises an alignment arm (130447) configured to be received within a corresponding alignment aperture (130415) defined in said second jaw (10410, 110410, 130410, 140410).

3. The surgical stapling assembly of Claim 1, wherein said coupling members are recessed relative to an outer surface of said second jaw (10410, 110410, 130410, 140410).

4. The surgical stapling assembly of Claim 1, wherein each said coupling member comprises a retaining clip (130440, 131440), wherein each said retaining clip comprises laterally extending alignment flanges (131447) configured to be received within corresponding alignment recesses (131447) defined within said second jaw.

5. The surgical stapling assembly of Claim 4, wherein each said alignment flange comprises a pin (131446) extending therefrom configured to be received within corresponding pin apertures (131416) defined in said alignment recesses.

6. The surgical stapling assembly of Claim 1, wherein each said coupling member comprises a circular body, and wherein each said recess comprises a circular shape configured to receive said circular body therein.

7. The surgical stapling assembly of Claim 1, wherein said first jaw (10420, 110420, 130420, 140420) is pivotable relative to said second jaw (10410, 110410, 130410, 140410).

8. The surgical stapling assembly of Claim 1, wherein said second jaw (10410, 110410, 130410, 140410) is pivotable relative to said first jaw (10420, 110420, 130420, 140420).

9. A method for manufacturing a surgical end effector assembly, comprising:
providing an anvil jaw (10420, 110420, 130420, 140420) comprising a pair of anvil trunnions (130425);
providing a cartridge jaw (10410, 110410, 130410, 140410) comprising a pair of anvil trunnion slots (130415);
providing coupling members (110720, 130440, 131440); and
assembling said anvil jaw to said cartridge jaw by:
inserting said anvil trunnions (130425) into said anvil trunnion slots (130415); and
placing said coupling members over said anvil trunnions within corresponding recesses (130417, 131417) defined in said cartridge jaw; **characterized by**
welding said coupling members to said cartridge jaw; and wherein
the anvil slots extend partially through said recesses.

## Patentansprüche

1. Chirurgische Klammeranordnung (151000, 152000, 152500), umfassend:
ein Klammermagazin (10430, 130430, 140430), umfassend einen Längsschlitz und eine Vielzahl von Klammern, die innerhalb des Klammermagazins entnehmbar gelagert sind,
eine erste Backe (10420, 110420, 130420, 140420), umfassend Kopplungsstifte (130425), die sich von einem proximalen Ende (130421) der ersten Backe (10420, 110420, 130420, 140420) seitlich nach außen erstrecken;
eine zweite Backe (10410, 110410, 130410, 140410), wobei eine der ersten Backe und der zweiten Backe relativ zu der anderen der ersten Backe und der zweiten Backe verschiebbar ist und wobei die zweite Backe umfasst:
Aussparungen (130417, 131417), die in der zweiten Backe definiert sind; und
Schlitze (130415), die in der zweiten Backe definiert sind, die konfiguriert sind, um die Kopplungsstifte (130425) aufzunehmen, um die erste Backe und die zweite Backe schwenkbar zu koppeln; und
Kopplungselemente (110720, 130440, 131440), die innerhalb der Aussparungen positioniert sind, wobei die Kopplungsstifte derart innerhalb von Schwenköffnungen (10415) aufgenommen sind, die in den Kopplungselementen definiert sind, dass die Kopplungselemente eine Translation zwischen der ersten Backe und der zweiten Backe derart beschränken, dass die einzige Bewegung zwischen der ersten Backe und der zweiten Backe eine Rotation um eine Schwenkachse herum umfasst, die durch die Kopplungsstifte und die Schwenköffnungen definiert ist; und **dadurch gekennzeichnet, dass**
die Schlitze, die in der zweiten Backe definiert sind, sich teilweise durch die Aussparungen erstrecken; und die Kopplungselemente mit der zweiten Backe verschweißt sind.

2. Chirurgische Klammeranordnung nach Anspruch 1, wobei jedes der Kopplungselemente einen Ausrichtungsarm (130447) umfasst, der konfiguriert ist, um innerhalb einer entsprechenden Ausrichtungsöffnung (130415) aufgenommen zu werden, die in der zweiten Backe (10410, 110410, 130410, 140410) definiert ist.

3. Chirurgische Klammeranordnung nach Anspruch 1, wobei die Kopplungselemente relativ zu einer Außenoberfläche der zweiten Backe (10410, 110410, 130410, 140410) ausgespart sind.

4. Chirurgische Klammeranordnung nach Anspruch 1, wobei jedes der Kopplungselemente einen Haltebügel (130440, 131440) umfasst, wobei jeder der Haltebügel sich seitlich erstreckende Ausrichtungsflansche (131447) umfasst, die konfiguriert sind, um innerhalb der entsprechenden Ausrichtungsaussparungen (131447) aufgenommen zu werden, die innerhalb der zweiten Backe definiert sind.

5. Chirurgische Klammeranordnung nach Anspruch 4, wobei jeder der Ausrichtungsflansche einen Stift (131446) umfasst, der sich davon erstreckt, der konfiguriert ist, um innerhalb von entsprechenden Stiftöffnungen (131416) aufgenommen zu werden, die in den Ausrichtungsaussparungen definiert sind.

6. Chirurgische Klammeranordnung nach Anspruch 1, wobei jedes der Kopplungselemente einen kreisförmigen Körper umfasst und wobei jede der Aussparungen eine kreisförmige Form umfasst, die konfiguriert ist, um den kreisförmigen Körper darin aufzunehmen.

7. Chirurgische Klammeranordnung nach Anspruch 1, wobei die erste Backe (10420, 110420, 130420, 140420) relativ zu der zweiten Backe (10410, 110410, 130410, 140410) schwenkbar ist.

8. Chirurgische Klammeranordnung nach Anspruch 1, wobei die zweite Backe (10410, 110410, 130410, 140410) relativ zu der ersten Backe (10420, 110420, 130420, 140420) schwenkbar ist.

9. Verfahren zum Herstellen einer chirurgischen Endeffektoranordnung, umfassend:
Bereitstellen einer Ambossbacke (10420, 110420, 130420, 140420), umfassend ein Paar von Ambosszapfen (130425);
Bereitstellen einer Magazinbacke (10410, 110410, 130410, 140410), umfassend ein Paar von Ambosszapfenschlitzen (130415);
Bereitstellen der Kopplungselemente (110720, 130440, 131440); und
Anordnen der Ambossbacke mit der Magazinbacke durch:
Einsetzen der Ambosszapfen (130425) in die Ambosszapfenschlitze (130415); und
Platzieren der Kopplungselemente über den Ambosszapfen innerhalb der entsprechenden Aussparungen (130417, 131417), die in der Magazinbacke definiert sind; **gekennzeichnet durch**
Verschweißen der Kopplungselemente mit der Magazinbacke; und wobei sich die Ambossschlitze teilweise durch die Aussparungen erstrecken.

## Revendications

1. Ensemble d'agrafage chirurgical (151000, 152000, 152500), comprenant :
une cartouche d'agrafes (10430, 130430, 140430) comprenant une fente longitudinale et une pluralité d'agrafes stockées de manière amovible à l'intérieur de ladite cartouche d'agrafes,
une première mâchoire (10420, 110420, 130420, 140420) comprenant des tiges d'accouplement (130425) s'étendant latéralement vers l'extérieur à partir d'une extrémité proximale (130421) de ladite première mâchoire (10420, 110420, 130420, 140420) ;
une seconde mâchoire (10410, 110410, 130410, 140410), dans lequel l'une parmi ladite première mâchoire et ladite seconde mâchoire est mobile par rapport à l'autre parmi ladite première mâchoire et ladite seconde mâchoire, et dans lequel ladite seconde mâchoire comprend :
des évidements (130417, 131417) définis dans ladite seconde mâchoire ; et
des fentes (130415) définies dans ladite seconde mâchoire qui sont conçues pour recevoir lesdites tiges d'accouplement (130425) afin d'accoupler de manière pivotante ladite première mâchoire et ladite seconde mâchoire ; et
des éléments d'accouplement (110720, 130440, 131440) positionnés à l'intérieur desdits évidements, dans lequel lesdites tiges d'accouplement sont reçues à l'intérieur d'ouvertures de pivotement (10415) définies dans lesdits éléments d'accouplement de telle sorte que lesdits éléments d'accouplement limitent la translation entre ladite première mâchoire et ladite seconde mâchoire de telle sorte que le seul mouvement entre ladite première mâchoire et ladite seconde mâchoire comprend une rotation autour d'un axe de pivotement défini par lesdites tiges d'accouplement et lesdites ouvertures de pivotement ; et **caractérisé en ce que**
les fentes définies dans la seconde mâchoire s'étendent partiellement à travers les évidements ; et les éléments d'accouplement sont soudés à la seconde mâchoire.

2. Ensemble d'agrafage chirurgical selon la revendication 1, dans lequel chaque dit élément d'accouplement comprend un bras d'alignement (130447) conçu pour être reçu à l'intérieur d'une ouverture d'alignement (130415) correspondante définie dans ladite seconde mâchoire (10410, 110410, 130410, 140410).

3. Ensemble d'agrafage chirurgical selon la revendication 1, dans lequel lesdits éléments d'accouplement sont en retrait par rapport à une surface externe de ladite seconde mâchoire (10410, 110410, 130410, 140410).

4. Ensemble d'agrafage chirurgical selon la revendication 1, dans lequel chaque dit élément d'accouplement comprend un clip de retenue (130440, 131440), dans lequel chaque clip de retenue comprend des brides d'alignement s'étendant latéralement (131447) conçues pour être reçues à l'intérieur d'évidements d'alignement (131447) correspondants définis à l'intérieur de ladite seconde mâchoire.

5. Ensemble d'agrafage chirurgical selon la revendication 4, dans lequel chaque dite bride d'alignement comprend une tige (131446) s'étendant à partir de celle-ci, conçue pour être reçue à l'intérieur d'ouvertures de tige (131416) correspondantes définies à l'intérieur desdits évidements d'alignement.

6. Ensemble d'agrafage chirurgical selon la revendication 1, dans lequel chaque dit élément d'accouplement comprend un corps circulaire, et dans lequel chaque dit évidement comprend une forme circulaire conçue pour recevoir ledit corps circulaire à l'intérieur de celui-ci.

7. Ensemble d'agrafage chirurgical selon la revendication 1, dans lequel ladite première mâchoire (10420, 110420, 130420, 140420) peut pivoter par rapport à ladite seconde mâchoire (10410, 110410, 130410, 140410).

8. Ensemble d'agrafage chirurgical selon la revendication 1, dans lequel ladite seconde mâchoire (10410, 110410, 130410, 140410) peut pivoter par rapport à ladite première mâchoire (10420, 110420, 130420, 140420).

9. Procédé de fabrication d'un ensemble effecteur chirurgical, comprenant :
la fourniture d'une mâchoire d'enclume (10420, 110420, 130420, 140420) comprenant une paire de tourillons d'enclume (130425) ;
la fourniture d'une mâchoire de cartouche (10410, 110410, 130410, 140410) comprenant une paire de fentes de tourillon d'enclume (130415) ;
la fourniture d'éléments d'accouplement (110720, 130440, 131440) ; et
l'assemblage de ladite mâchoire d'enclume à ladite mâchoire de cartouche par :
l'insertion desdits tourillons d'enclume (130425) dans lesdites fentes de tourillons d'enclume (130415) ; et
le placement desdits éléments d'accouplement par-dessus lesdits tourillons d'enclume à l'intérieur d'évidements (130417, 131417) correspondants définis dans ladite mâchoire de cartouche ; **caractérisé par**
le soudage desdits éléments d'accouplement à ladite mâchoire de cartouche ; et dans lequel les fentes d'enclume s'étendent partiellement à travers lesdits évidements.
